# EUROPÄISCHE PATENTSCHRIFT

(11) **EP 4 186 543 B1**
(45) Veröffentlichungstag und Bekanntmachung des Hinweises auf die Patenterteilung: **20.11.2024**
(21) Anmeldenummer: 21211357.5
(22) Anmeldetag: 30.11.2021
(51) Int. Cl.: A61M 5/162, A61M 5/32, A61M 5/34, A61M 5/158, C12M 1/12, A61M 39/16

(54) **VERFAHREN ZUR STERILEN KONNEKTIERUNG ZWEIER GESCHLOSSENER SYSTEME MITHILFE VON HEISSSTERILISIERUNG**
METHOD FOR STERILE CONNECTION OF TWO CLOSED SYSTEMS USING HOT STERILIZATION
PROCÉDÉ DE CONNEXION STÉRILE DE DEUX SYSTÈMES FERMÉS PAR STÉRILISATION À LA CHALEUR

(43) Veröffentlichungstag der Anmeldung: 31.05.2023
(73) Patentinhaber: Fraunhofer-Gesellschaft zur Förderung der angewandten Forschung e.V., 80686 München (DE)
(72) Erfinder: Pfeifer, Michael, 70569 Stuttgart (DE); Schandar, Markus, 70569 Stuttgart (DE)
(74) Vertreter: Schrell, Andreas

(56) Entgegenhaltungen:
- JP-A- 2007 300 814
- JP-A- 2008 220 307
- US-A1- 2016 279 326
- US-A1- 2017 354 571
- US-A1- 2020 340 606
- US-B2- 7 703 486

## Beschreibung

Die vorliegende Erfindung betrifft Sterilkonnektoren zur sterilen Verbindung eines ersten geschlossenen Systems mit einem zweiten geschlossenen System, Verfahren zum sterilen Verbinden eines ersten geschlossenen Systems mit einem zweiten geschlossenen System mittels eines Sterilkonnektors sowie Vorrichtungen zur Verbindung eines ersten geschlossenen Systems mit einem Sterilkonnektor.

Die sterile Konnektierung/Verbindung ist ein Vorgang, bei dem unter Wahrung der Sterilität eine Verbindung zwischen zwei geschlossenen Systemen geschaffen wird, welche den Transfer eines Mediums, meist einer Flüssigkeit erlaubt. Ein geschlossenes System ist vor allem charakterisiert dadurch, dass es ohne direkten menschlichen Eingriff keine Materie mit der Umgebung austauschen kann. Bei der Produktion von modernen medizinischen Therapeutika werden unter anderem Zellen gentechnisch verändert und in einem Wachstumsmedium inkubiert. Hierzu müssen im Prozess unterschiedliche Medien (wie zum Beispiel virale Vektoren, Wachstumsfaktoren, Enzymlösungen, und Ähnliches) hinzugefügt werden. Da derartige Medien aus verschiedenen Gründen (zum Beispiel Kühlung) nicht von Beginn an Teil eines geschlossenen Systems sein können, müssen diese später aus einem anderen sterilen Behältnis heraus hinzugefügt werden. Da ein einzelner Keim diesen aufwändigen Prozess gefährden kann, ist Sterilität eine unverzichtbare Grundvoraussetzung für jegliche Konnektierung derartiger geschlossener Systeme. Derzeit werden medizinische Glasfläschchen mit Gummiverschluss ("Vials"), die ein gewünschtes Medium enthalten, mittels eines hohlen Dorns angestochen und entleert. Im Zusammenhang mit aseptischen Produktionsverfahren muss dies allerdings unter sterilen Umgebungsbedingungen erfolgen, das heißt in einer Sterilbank (Klasse A) in einem Reinraum (Klasse B).

Geschlossene Systeme, insbesondere geschlossene Anlagen, in der Pharmaproduktion müssen wegen des Anschließens flüssiger Komponenten während laufender Produktion in qualifizierten Reinräumen (Klasse A, B) aufgestellt oder transportiert werden, da das Anstechen nicht als geschlossener Prozessschritt gilt. Reinräume sind nicht nur in der Erstellung, sondern auch im Betrieb extrem teuer. Das Arbeiten in Reinräumen stellt hohe Anforderungen an das Personal und ist wesentlich zeitintensiver als in sonstigen Produktionsräumen.

Einschlägige Sterilkonnektoren zur sterilen Verbindung eines ersten geschlossenen Systems sind aus der US 2020/340606 A1 bekannt.

Der vorliegenden Erfindung liegt daher das technische Problem zugrunde, die Nachteile des Standes der Technik zu überwinden, insbesondere einen Sterilkonnektor zur Verfügung zu stellen, welcher es ermöglicht, zwei geschlossene System, ohne das ein Reinraum und die damit verbundenen Kosten nötig sind, zu verbinden. Insbesondere liegt der vorliegenden Erfindung das technische Problem zugrunde ein erstes geschlossenes System mit einem sterilen Sterilkonnektor, ohne dass das geschlossene System kompromittiert wird, das heißt Keime von außerhalb des geschlossenen Systems in dieses gelangen, zu verbinden und somit insbesondere regulatorischen Anforderungen, wie zum Beispiel GMP, gerecht zu werden.

Die vorliegende Erfindung löst das ihr zugrundeliegende technische Problem durch die Bereitstellung eines Sterilkonnektors zur sterilen Verbindung eines ersten geschlossenen Systems mit einem zweiten geschlossenen System umfassend:
- mindestens eine Kanüle mit einem Einstich- und einem Anschlussbereich, wobei der Einstichbereich ein induktiv erhitzbares Material umfasst und mit einer elastisch verformbaren, erhitzungsresistenten Hülle aus Kunststoff ummantelt ist,
- ein fluiddicht mit dem Anschlussbereich der Kanüle verbundenes Anschlusselement, welches ausgebildet ist zur Herstellung einer Fluid-Verbindung zwischen der Kanüle und dem zweiten geschlossenen System,
- ein an der Kanüle angeordnetes, die Kanüle und das Anschlusselement fluiddicht verbindendes Verbindungselement, welches ausgebildet ist zur kraftschlüssigen oder integralen Verbindung mit einer Arretiervorrichtung, und
- eine mit dem Verbindungselement kraftschlüssig oder integral verbundene Arretiervorrichtung, umfassend ein Rahmenteil und mindestens eine Rastvorrichtung, wobei die Rastvorrichtung ausgebildet ist zur Arretierung des Sterilkonnektors an dem ersten geschlossenen System und wobei die Arretiervorrichtung den ummantelten Einstichbereich der Kanüle allseitig unter Bildung einer Anschlussöffnung für das erste geschlossene System umfasst, insbesondere ohne die Hülle der Kanüle zu kontaktieren.

Der erfindungsgemäße Sterilkonnektor ist so ausgebildet, dass er ein geschlossenes System ist, welches in seinem Inneren steril ist.

Der erfindungsgemäße Sterilkonnektor zeichnet sich dadurch aus, dass er mindestens eine, bevorzugt eine, Kanüle, ein Verbindungselement, ein Anschlusselement und eine Arretiervorrichtung umfasst. Das Verbindungselement verbindet die Kanüle, das Anschlusselement und die Arretiervorrichtung, wobei die Kanüle und das Anschlusselement an gegenüberliegenden Enden des Verbindungselements mit diesem verbunden sind und die Arretiervorrichtung mit dem Verbindungselement kraftschlüssig oder integral, das bedeutet einstückig, so verbunden ist, dass diese allseitig die ummantelte Kanüle umfasst.

Die mindestens eine Kanüle umfasst einen Einstichbereich und einen Anschlussbereich. Der Einstichbereich ist dabei ein, vom Verbindungselement gesehen, distaler Bereich an der Kanüle, welcher die Verbindung mit dem ersten geschlossenen System herstellt, indem dieser Bereich in das erste geschlossene System eingestochen wird. Der Anschlussbereich ist ein Bereich proximal zum Verbindungselement und ist direkt mit dem Verbindungselement verbunden. Alle drei Elemente, Kanüle, Verbindungselement und Anschlusselement, sind auf eine Weise verbunden, insbesondere fluiddicht verbunden, sodass sie eine Fluid-Verbindung herstellen können zwischen einem ersten geschlossenen System und einem zweiten geschlossenen System. Um die Sterilität des Sterilkonnektors zu gewährleisten, ist die Kanüle dabei mit einer Hülle aus Kunststoff ummantelt. Die Hülle selbst besteht aus einem Material, welches elastisch verformbar ist, um eine Pfropfbildung in der Kanüle beim Einstechen in das erste geschlossene System zu verhindern. Die Hülle verhindert insbesondere, dass Keime direkt an der Kanüle anhaften und so in das erste geschlossene System übertragen werden könnten. Ferner ist die Hülle erhitzungsresistent, um zu verhindern, dass die Hülle schmilzt oder ihre Form verändert, während die Kanüle erhitzt wird. Um die Erhitzung der Kanüle zu ermöglichen, umfasst diese induktiv erhitzbares Material.

Der Sterilkonnektor umfasst ferner eine Arretiervorrichtung, welche kraftschlüssig oder integral mit dem Verbindungselement verbunden ist. Diese Arretiervorrichtung umfasst einen Rahmenteil sowie mindestens eine Rastvorrichtung. Die Rastvorrichtung ist dazu ausgebildet, den Sterilkonnektor an dem ersten geschlossenen System zu arretieren, um so insbesondere zu verhindern, dass die Kanüle sich seitlich innerhalb des und/oder aus dem ersten geschlossenen System bewegt, daher beide stabil miteinander verbunden sind. Ebenso kann die Arretiervorrichtung so ausgelegt sein, dass sie verhindert, dass die Kanüle zu tief in das erste geschlossene System eindringt, insbesondere in eine Flüssigkeit in dem ersten geschlossenen System, und so, da die Kanüle nach Erhitzung sehr heiß sein kann, den Inhalt beschädigt oder zerstört. Die Arretiervorrichtung umfasst dabei den ummantelten Einstichbereich der Kanüle allseitig unter Bildung einer Anschlussöffnung, welche dazu dient, das erste geschlossene System aufzunehmen. Die allseitige Umfassung ist dabei so ausgestaltet, dass diese nicht das vom Verbindungselement aus gesehene distale Ende der Kanüle abdeckt, welches für den Einstich und die Verbindung des Sterilkonnektors mit dem ersten geschlossenen System notwendig ist. Vielmehr wird bei der Umfassung eine Anschlussöffnung gebildet, welche es ermöglicht, dass die Kanüle in das erste geschlossene System eingestochen werden kann und die Arretiervorrichtung das erste geschlossene System aufnehmen und arretieren kann. Die Anschlussöffnung ist dabei auf die Geometrie des ersten geschlossenen Systems abgestimmt, wobei diese nicht zwangsläufig eine runde Form aufweisen muss. In bevorzugter Weise wird dabei die Hülle durch die Arretiervorrichtung nicht kontaktiert.

Der Vorteil eines erfindungsgemäßen Sterilkonnektors liegt darin, dass dieser, insbesondere mithilfe einer geeigneten Vorrichtung zur Erhitzung der Kanüle, verwendet werden kann, um zwei geschlossene, insbesondere sterile, Systeme miteinander über eine Fluid-Verbindung zu verbinden, ohne dass hierfür ein spezieller Reinraum notwendig ist. Die Verbindung kann stattdessen unter nicht sterilen Bedingungen erfolgen, wobei, da der Sterilkonnektor in seinem Inneren selbst steril ist, die geschlossenen Systeme und die Verbindung zwischen ihnen nicht kompromittiert werden, das heißt insbesondere kein Keim in diese eindringen kann.

In einer bevorzugten Ausführungsform ist der Einstichbereich zum offenen, vom Verbindungselement aus distalem Ende, hin spitz zulaufend, um das Einstechen in das erste geschlossene System zu erleichtern.

In einer bevorzugten Ausführungsform ist die Kanüle zumindest teilweise aus Metall als induktiv erhitzbares Material gefertigt, insbesondere aus einer Mischung von Nickel, Chrom und Stahl.

In einer bevorzugten Ausführungsform ist der Einstichbereich der Kanüle zumindest teilweise aus Metall als induktiv erhitzbares Material gefertigt, insbesondere aus einer Mischung von Nickel, Chrom und Stahl.

In einer bevorzugten Ausführungsform besteht der Einstichbereich der Kanüle aus Metall als induktiv erhitzbares Material, insbesondere aus einer Mischung von Nickel, Chrom und Stahl.

In einer bevorzugten Ausführungsform ist der Anschlussbereich der Kanüle zumindest teilweise aus Kunststoff gefertigt, insbesondere ein mittels Gammastrahlung sterilisierbarer und/oder autoklavierbarer Kunststoff, insbesondere Polypropylen.

In einer bevorzugten Ausführungsform besteht der Anschlussbereich der Kanüle aus Kunststoff, insbesondere Polypropylen.

In einer bevorzugten Ausführungsform besteht die Kanüle aus zwei Abschnitten unterschiedlicher Wärmekapazität.

In einer bevorzugten Ausführungsform weisen der Einstichbereich und der Anschlussbereich der Kanüle unterschiedliche Wärmekapazitäten auf.

In einer bevorzugten Ausführungsform weist der Einstichbereich eine spezifische Wärmekapazität von 400 bis 500 J/(kg*K) auf und besteht vorzugsweise aus einem induktiv erhitzbaren Material, insbesondere Metall, insbesondere aus einer Mischung von Nickel, Chrom und Stahl.

In einer bevorzugten Ausführungsform weist der Anschlussbereich eine spezifische Wärmekapazität von 1500 bis 1900 J/(kg*K) auf und besteht vorzugsweise aus Kunststoff, insbesondere Polypropylen.

Eine erhöhte Wärmekapazität insbesondere des Anschlussbereiches im Vergleich zum Einstichbereich sorgt dafür, dass die Erhitzung des Einstichbereichs bei gleicher eingetragener Energie schneller erfolgt als im Anschlussbereich.

In einer bevorzugten Ausführungsform besteht die elastisch verformbare, erhitzungsresistente Hülle aus Kunststoff, welcher bis zu einer Temperatur von 300 °C nicht schmilzt oder seine molekulare Struktur zerstört wird.

In einer bevorzugten Ausführungsform besteht die elastisch verformbare, erhitzungsresistente Hülle aus Kunststoff, insbesondere Kunststoff, welcher für Medizinprodukte geeignet ist, insbesondere aus einem Elastomer, insbesondere Gummi oder Silikon. In bevorzugter Ausführungsform besteht die Hülle nicht aus einem thermoplastischen Kunststoff. Ein thermoplastischer Kunststoff kann bei Erhitzung zur Pfropfbildung beim Einstechen in das erste geschlossene System führen.

In einer bevorzugten Ausführungsform ist das Anschlusselement ein Schlauch, Rohr, Kanal oder sonstiges zur fluiddichten Verbindung geeignetes Verbindungssystem, insbesondere ein Kunststoff-Schlauch, insbesondere ein Polyvinylchlorid(PVC)-Schlauch, welcher bevorzugt an dem der Kanüle abgewandten Ende verschlossen ist.

In einer bevorzugten Ausführungsform ist das Anschlusselement ein Beutel.

In einer bevorzugten Ausführungsform ist das Anschlusselement ein Element beliebiger Komplexität.

In einer bevorzugten Ausführungsform ist das Anschlusselement ein geschlossenes Verbindungssystem, insbesondere von beliebiger Komplexität.

In einer bevorzugten Ausführungsform ist an das Anschlusselement, am distalen Ende (gesehen vom Verbindungselement) eine, insbesondere gasgefüllte, insbesondere steriler Luft, Spritze angebracht, insbesondere in fluiddichter Verbindung.

In einer bevorzugten Ausführungsform ist die Arretiervorrichtung, insbesondere die Rastvorrichtung der Arretiervorrichtung, zur Arretierung an ein Gefäß oder ein Raum- oder Gefäßsystem umfassend fluiddicht und steril miteinander verbundene Bereiche, insbesondere Räume, insbesondere Gefäße, insbesondere ein System beliebiger Komplexität, ausgebildet.

In einer bevorzugten Ausführungsform ist die Arretiervorrichtung, insbesondere die Rastvorrichtung der Arretiervorrichtung, zur Arretierung an ein Gefäß, insbesondere eine Bördelflasche aus Glas oder Plastik, ein Zellkulturbeutel oder Blutbeutel, oder ein beliebig komplexes geschlossenes System mit einem Septum, insbesondere aus einem Elastomer, insbesondere Gummi oder Silikon, ausgebildet.

In einer bevorzugten Ausführungsform weist die Rastvorrichtung der Arretiervorrichtung Mittel auf, die einrasten, sobald die Kanüle in einer gewünschten Länge in das erste geschlossene System eingeführt ist, und das erste geschlossene System so arretieren, dass die Kanüle nicht mehr innerhalb, insbesondere seitlich innerhalb, des ersten geschlossenen Systems bewegt werden kann. Diese Arretierung kann zusätzlich verhindern, dass es zu einer möglichen Kompromittierung der Verbindung oder der geschlossenen Systeme durch Bewegungen der Kanüle kommt. Ebenso kann verhindert werden, dass die Kanüle zu tief in das geschlossene System eindringt und, falls die Kanüle erhitzt wurde, so den Inhalt des ersten geschlossenen Systems teilweise oder vollständig zerstört.

In einer bevorzugten Ausführungsform weist die Arretiervorrichtung Mittel auf, welche verhindern, dass der Sterilkonnektor von dem ersten geschlossenen System nach Verbindung des Sterilkonnektors mit dem ersten geschlossenen System getrennt werden kann, ohne dass der Sterilkonnektor oder das erste geschlossene System beschädigt werden.

In einer bevorzugten Ausführungsform weist die Arretiervorrichtung Öffnungen auf, welche es ermöglichen einen Gasstrom auf die Hülle um die Kanüle und/oder dem Verbindungselement zu richten, um diese bei einer Erhitzung der Kanüle zu kühlen.

In einer bevorzugten Ausführungsform weist der Sterilkonnektor eine Kanüle auf.

In einer bevorzugten Ausführungsform weist der Sterilkonnektor mindestens zwei, insbesondere zwei, Kanülen auf, wobei beide Kanülen von einer gemeinsamen Hülle ummantelt sind und mit ihren Einstichbereichen in das erste geschlossene System eingeführt werden können. Die erste Kanüle stellt dabei über das Anschlusselement eine Fluid-Verbindung zwischen erstem geschlossenem System und dem zweiten geschlossenen System bereit. Jede weitere, insbesondere zweite, Kanüle weist einen eigenen Anschlussbereich auf, welcher über einen Sterilfilter zur Umgebung oder mit einem weiteren geschlossenen System in Verbindung steht.

In einer bevorzugten Ausführungsform weist der Sterilkonnektor eine Kanüle mit mindestens zwei, insbesondere mindestens drei, insbesondere mindestens vier, bevorzugt zwei, bevorzugt drei, bevorzugt vier, Kanälen auf. In dieser bevorzugten Ausführungsform ist die Kanüle mit den mindestens zwei Kanälen umhüllt. Der erste Kanal stellt dabei eine Fluid-Verbindung zwischen dem ersten geschlossenen System und dem zweiten geschlossenen System über das Anschlusselement bereit. Jeder weitere, insbesondere zweite, Kanal weist einen eigenen separaten Anschlussbereich auf, welcher über einen Sterilfilter zur Umgebung oder mit einem weiteren geschlossenen System in Verbindung steht.

In einer bevorzugten Ausführungsform ist das mindestens eine weitere geschlossene System ein gasgefüllter, insbesondere mit steriler Luft oder mit einem Schutzgas, insbesondere Argon und/oder Stickstoff, gefüllter, verformbarer Beutel, vorzugsweise aus Kunststoff.

In einer bevorzugten Ausführungsform ist das mindestens eine weitere geschlossene System eine Spritze. Diese kann vorzugsweise so angeschlossen werden, dass sie, nachdem der Kolben eingedrückt wurde, abgenommen wird, und erneut mit, insbesondere steriler, Luft gefüllt werden kann, wobei die Spritze vorzugsweise über einen Sterilfilter an die weitere Kanüle oder Kanal angeschlossen ist.

Die Ausführungsformen mit mindestens zwei Kanülen oder einer Kanüle mit mindestens zwei Kanälen bieten den Vorteil, dass ein Unterdruck, welcher durch den Abfluss des Inhalts, insbesondere einer Flüssigkeit, aus dem ersten in das zweite geschlossene System entsteht, über die zweite Kanüle oder den zweiten Kanal ausgeglichen werden kann oder dass ein Überdruck in dem ersten geschlossenen System aufgebaut werden kann, um den Fluss zum Beispiel einer Flüssigkeit aus dem ersten in das zweite geschlossene System zu unterstützen, insbesondere zu beschleunigen. Ebenso kann im Falle eines Überdrucks im ersten geschlossenen System dieser mithilfe einer weiteren Kanüle oder Kanals ausgeglichen werden, um die Dichtigkeit und damit die Geschlossenheit des geschlossenen Systems zu unterstützen.

Ohne an die Theorie gebunden zu sein, kann ein Unterdruck in einem zum Beispiel mit einer Flüssigkeit gefüllten ersten geschlossenen System dazu führen, dass das System undicht, daher nicht mehr geschlossen, ist. Zum Beispiel wenn das erste geschlossene System ein Septum aus Silikon aufweist und die Kanüle des Sterilkonnektors in dieses eingestochen ist, kann es passieren, dass das Septum, aufgrund eines Unterdrucks, die Kanüle nicht mehr vollständig umschließt, und somit seine Geschlossenheit verliert, weil Luft von außen in das erste System eindringt. Ein Überdruck selbst würde nicht zu einer Kontamination führen, jedoch eventuell zu einem Verlust von zum Beispiel Flüssigkeit aus dem ersten geschlossenen System und somit zu einem Verlust einer zuvor bestimmten Dosis und somit zu einer ungenauen Dosierung.

Die Erfindung betrifft ebenso Verfahren zum sterilen Verbinden eines ersten geschlossenen Systems mit einem zweiten geschlossenen System umfassend die folgenden Verfahrensschritte:
a) Bereitstellen eines ersten und eines zweiten geschlossenen Systems, eines erfindungsgemäßen Sterilkonnektors und einer Vorrichtung zur Erhitzung der mindestens einen Kanüle des Sterilkonnektors,
b) Erhitzen mindestens eines Abschnitts der mindestens einen Kanüle auf mindestens 60 °C, für eine Zeit, die ausreicht, um den mindestens einen Abschnitt zu sterilisieren,
c) Verbinden des Sterilkonnektors mit dem ersten geschlossenen System durch Einführen der mindestens einen Kanüle in das erste geschlossene System,
d) Verbinden des Sterilkonnektors mit dem zweiten geschlossenen System mit dem Anschlusselement, wodurch eine Fluid-Verbindung zwischen dem ersten und zweiten geschlossenen System durch den Sterilkonnektor hergestellt wird,
wobei vorzugsweise Verfahrensschritte b) und c) zumindest teilweise zeitlich parallel ablaufen oder nacheinander und wobei Verfahrensschritt d) vor Verfahrensschritt b) oder nach Verfahrensschritt c) durchgeführt wird.

Das erfindungsgemäße Verfahren bietet unter anderem den Vorteil, dass durch die Kombination aus erfindungsgemäßem Sterilkonnektor, daher vor allem einem Sterilkonnektor, dessen Kanüle mit einer Hülle ummantelt ist, und Erhitzung, insbesondere mittels Induktion, die ohne die Hülle zu öffnen abläuft, die Wahrscheinlichkeit einer Kontamination in erheblichem Maß verringert, da keine, insbesondere vermehrungsfähigen, Keime von der Hülle oder von der Oberfläche des ersten geschlossenen Systems in den Sterilkonnektor oder das erste geschlossenen System eindringen können. Insbesondere die Erhitzung sorgt dafür, dass eventuell vorhandene Keime, insbesondere auf der Oberfläche des ersten geschlossenen Systems abgetötet werden.

In einer bevorzugten Ausführungsform ist das erste geschlossene System ein Gefäß oder ein Raum- oder Gefäßsystem umfassend fluiddicht und steril miteinander verbundene Bereiche, insbesondere Räume, insbesondere Gefäße, insbesondere ein System beliebiger Komplexität.

In einer bevorzugten Ausführungsform ist das erste geschlossene System ein Gefäß, welches über ein Septum verschlossen ist. Vorzugsweise besteht das Septum aus einem Elastomer, insbesondere Gummi oder Silikon.

In einer bevorzugten Ausführungsform ist das erste geschlossene System eine Bördelflasche aus Glas oder Plastik, ein Zellkulturbeutel oder ein Blutbeutel. Das erste geschlossene System weist vorzugsweise eine beliebige Komplexität auf.

In einer bevorzugten Ausführungsform ist das erste geschlossene System ein Gefäß, welches über ein Septum verschlossen ist, und wobei das Verbinden in Verfahrensschritt c) durch Einstechen der mindestens einen Kanüle des Sterilkonnektors in das Septum erfolgt. Wenn die Kanüle das Septum des ersten geschlossenen Systems durchstochen hat, dichtet das Septum die Verbindung an der Kanüle ab.

In einer bevorzugten Ausführungsform herrscht im ersten geschlossenen System, welches in Verfahrensschritt a) bereitgestellt wird, ein Überdruck. Dieser Überdruck kann helfen, die Geschlossenheit bei der Verbindung zwischen dem ersten und zweiten geschlossenen System über den Sterilkonnektor zu unterstützen, indem ein Unterdruck, welcher beim Übergang des Inhalts, insbesondere einer Flüssigkeit, vom ersten in das zweite geschlossene System entsteht, verringert wird. In bevorzugter Ausführungsform ist eine gasgefüllte, insbesondere mit steriler Luft, Spritze am Anschlusselement des Sterilkonnektors angebracht, welche dazu dient, nach dem Verbinden in Verfahrensschritt b) einen Überdruck im ersten geschlossenen System zu erzeugen.

In einer bevorzugten Ausführungsform weist das zweite geschlossene System einen Schlauch, insbesondere einen Kunststoffschlauch, insbesondere einen PVC-Schlauch, auf, über welchen in Verfahrensschritt d) der Sterilkonnektor mit dem zweiten geschlossenen System verbunden wird, bevorzugt mittels sterilem Schlauchschweißen.

In einer bevorzugten Ausführungsform ist das zweite geschlossene System ein Gefäß oder ein Raum- oder Gefäßsystem umfassend fluiddicht und steril miteinander verbundene Bereiche, insbesondere Räume, insbesondere Gefäße, insbesondere ein System beliebiger Komplexität.

Vorzugsweise ist das zweite geschlossene System ein Produktionssystem in der medizinischen und/oder biotechnologischen Produktion, insbesondere ein Bioreaktor.

In einer bevorzugten Ausführungsform ist der Sterilkonnektor vor dem Verbinden in Verfahrensschritt b) mit dem zweiten geschlossenen System verbunden, insbesondere in einem Verfahrensschritt d), wobei beide Verfahrensschritte sowohl räumlich als auch zeitlich getrennt ablaufen können, daher Verfahrensschritt d) kann gegebenenfalls mehrere Monate und an einem anderen Ort vor Verfahrensschritt b) stattgefunden haben.

In einer bevorzugten Ausführungsform wird in Verfahrensschritt b) die Kanüle mittels Induktion oder Strahlung, insbesondere Infrarotstrahlung, erhitzt. Der Vorteil, insbesondere der Erhitzung mittels Induktion, ist es, dass die Schutzhülle nicht unmittelbar zusätzlich erhitzt und somit vor allem vor Beschädigungen durch Verformen oder Schmelzen der Hülle geschützt ist.

In einer bevorzugten Ausführungsform wird in Verfahrensschritt b) mindestens ein Abschnitt der mindestens einen Kanüle auf mindestens 120 °C, insbesondere mindestens 140 °C, insbesondere mindestens 160 °C, insbesondere mindestens 180 °C, insbesondere 60 bis 300 °C, insbesondere 120 bis 300 °C, insbesondere 140 bis 300 °C, insbesondere 160 bis 300 °C, insbesondere 180 bis 300 °C erhitzt.

In einer bevorzugten Ausführungsform wird während Verfahrensschritt b) der Anschlussbereich der Kanüle und/oder das Verbindungselement des Sterilkonnektors mittels eines Gasstroms, insbesondere von Luft, insbesondere steriler Luft, insbesondere eines Schutzgases, insbesondere Argon und/oder Stickstoff, gekühlt. Dieses bietet den Vorteil, dass diese Bereiche/Elemente vor Beschädigungen durch Verformen oder Schmelzen geschützt sind, da diese abgekühlt werden.

In einer bevorzugten Ausführungsform wird in dem Verfahren als Vorrichtung zur Erhitzung der mindestens einen Kanüle des Sterilkonnektors eine Vorrichtung eingesetzt, welche eine Induktionsspule aufweist, welche dazu verwendet wird, in Verfahrensschritt b) die Kanüle/n des Sterilkonnektors zu erhitzen.

In einer bevorzugten Ausführungsform umfasst das Verfahren einen zusätzlichen Verfahrensschritt e), welcher zeitlich nach Verfahrensschritten b), c) und d) oder d), b) und c) durchgeführt wird, in welchen zumindest ein Teil des Inhalts, insbesondere einer Flüssigkeit, vom ersten geschlossenen System über den Sterilkonnektor in das zweite geschlossene System überführt wird.

In einer bevorzugten Ausführungsform umfasst das Verfahren zusätzlich einen Verfahrensschritt e) und einen Verfahrensschritt f), in welchem die Verbindung zwischen Sterilkonnektor und zweitem geschlossenen System getrennt wird, insbesondere durch ein Versiegeln des Anschlusselements und des zweiten geschlossenen Systems.

In einer bevorzugten Ausführungsform wird in dem Verfahren als Vorrichtung zur Erhitzung der mindestens einen Kanüle des Sterilkonnektors eine erfindungsgemäße Vorrichtung zur Verbindung eines ersten geschlossenen Systems mit einem Sterilkonnektor verwendet.

Die vorliegende Erfindung betrifft ebenso eine Vorrichtung zur, insbesondere automatisierten, Verbindung eines ersten geschlossenen Systems mit einem Sterilkonnektor, insbesondere in einem erfindungsgemäßen Verfahren, umfassend:
- Befestigung für ein erstes geschlossenes System
- eine kraftgetriebene mechanische Führung, an welcher ein erfindungsgemäßer Sterilkonnektor reversibel und kraftschlüssig fixierbar ist, und welche ausgebildet ist, den Sterilkonnektor in Fluid-Verbindung mit dem ersten geschlossenen System zu bringen, und
- Vorrichtung zum Erhitzen der mindestens einen Kanüle des Sterilkonnektors, insbesondere mittels Induktion oder Strahlung, insbesondere Infrarotstrahlung.

In einer bevorzugten Ausführungsform ist die Befestigung für ein erstes geschlossenes System so ausgelegt, dass dieses das erste geschlossene System fixiert, sodass dieses sich beim Verbinden mit dem Sterilkonnektor nicht bewegt.

In einer bevorzugten Ausführungsform umfasst die Vorrichtung zur Verbindung eines ersten geschlossenen Systems mit einem Sterilkonnektor als Vorrichtung zur Erhitzung eine Induktionsspule, insbesondere in Form einer Spirale oder eines Hufeisens, welche um die Arretiervorrichtung eines erfindungsgemäßen Sterilkonnektors angeordnet sein kann. Zur Erhitzung der mindestens einen Kanüle des Sterilkonnektors muss diese ein induktiv erhitzbares Material umfassen, bevorzugt ist die mindestens eine Kanüle mindestens teilweise aus Metall. Die Hufeisenform hat den Vorteil, dass der Sterilkonnektor leichter an der Vorrichtung angebracht und entfernt werden kann, jedoch den Nachteil, dass mehr Energie benötigt wird als eine Induktionsspule in Form einer Spirale.

In einer bevorzugten Ausführungsform ist die Induktionsspule in Form einer Spirale mechanisch klappbar gebaut, welche vor und nach dem Anbringen des Sterilkonnektors an die Vorrichtung geöffnet werden kann, um den Sterilkonnektor an der Vorrichtung anzubringen oder zu entfernen.

In einer bevorzugten Ausführungsform wird durch die Induktionsspule ein wechselndes Magnetfeld erzeugt, insbesondere in einem Bereich von 50 bis 800 kHz, insbesondere 50 bis 300 kHz.

In einer bevorzugten Ausführungsform weist die kraftgetriebene Führung der Vorrichtung zur Verbindung eines ersten geschlossenen Systems mit einem Sterilkonnektor einen beweglichen Schlitten auf, auf welcher der Sterilkonnektor reversibel und kraftschlüssig fixierbar ist. Vorzugsweise ist die Vorrichtung zur Erhitzung der Kanüle in dieser Ausführungsform so angeordnet, dass sie, während der Schlitten bewegt wird, die Kanüle erhitzen kann. Dies hat den Vorteil, dass die Kanüle bis kurz vor der Verbindung mit dem ersten geschlossen System erhitzt werden und somit die Geschlossenheit verbessert werden kann, indem sichergestellt wird, dass die Kanüle nicht frühzeitig abkühlt.

In einer bevorzugten Ausführungsform ist der Sterilkonnektor an der Vorrichtung zur Verbindung eines ersten geschlossenen Systems mit einem Sterilkonnektor mit einer Klammer reversibel und kraftschlüssig fixiert.

In einer bevorzugten Ausführungsform weist die Vorrichtung zur Verbindung eines ersten geschlossenen Systems mit einem Sterilkonnektor ferner eine Vorrichtung zur Kühlung von Teilen der Kanüle und/oder des Verbindungselements mittels Gasstroms, vorzugsweise Luft, vorzugsweise sterile Luft, auf. Vorzugsweise ist diese Vorrichtung zur Kühlung auf einem Schlitten der kraftgetriebenen mechanischen Führung angebracht.

In einer bevorzugten Ausführungsform weist die Vorrichtung zur Verbindung eines ersten geschlossenen Systems mit einem Sterilkonnektor ferner eine Steuereinheit auf, welche die Verbindung zwischen Sterilkonnektor und erstem geschlossenen System automatisiert durchführen kann. Insbesondere ist diese automatische Verbindung positions-, geschwindigkeits- und/oder kraftgesteuert. Die automatische Durchführung der Verbindung zwischen erstem geschlossenem System und Sterilkonnektor erhöht die Wahrscheinlichkeit, dass die Geschlossenheit des ersten geschlossenen Systems erhalten bleibt, während der Sterilkonnektor und das erste geschlossene System verbunden werden. Vor allem können dabei Hin- und Herbewegungen der Kanüle vermieden werden. Insbesondere wird durch die automatisierte Steuerung eine gleichmäßige Einstichgeschwindigkeit angewendet.

In einer bevorzugten Ausführungsform steuert die Steuereinheit die Erhitzung der Kanüle, insbesondere die Dauer der Erhitzung und/oder die Temperatur. Hierzu kann insbesondere die Energie, insbesondere die Menge und der Fluss, insbesondere die Dauer, gesteuert werden, welche für die Erhitzung aufgewendet wird.

In einer bevorzugten Ausführungsform steuert die Steuereinheit die Vorrichtung zur Kühlung, insbesondere den Gasstrom.

In einer bevorzugten Ausführungsform ist die Vorrichtung zur Verbindung eines ersten geschlossenen Systems mit einem Sterilkonnektor ein Handgerät, welches insbesondere also während des Prozesses gehalten werden kann. Dies hat insbesondere den Vorteil, dass durch die Handhabung sichergestellt werden kann, dass der Inhalt, insbesondere eine Flüssigkeit, im ersten geschlossenen System nicht in Kontakt mit der heißen Kanüle gerät, was zu einer zu schnellen Abkühlung der Kanüle führen könnte und zudem den Inhalt, insbesondere die Flüssigkeit, teilweise zerstören, insbesondere denaturieren, könnte, vor allem sollten im ersten geschlossenen System Proteine und/oder Nukleinsäuren, insbesondere virale Vektoren, enthalten sein.

In einer bevorzugten Ausführungsform weist die Vorrichtung zur Verbindung eines ersten geschlossenen Systems mit einem Sterilkonnektor einen erfindungsgemäßen Sterilkonnektor auf.

In einer bevorzugten Ausführungsform weist die Vorrichtung zur Verbindung eines ersten geschlossenen Systems mit einem Sterilkonnektor einen erfindungsgemäßen Sterilkonnektor und ein erstes geschlossenes System auf.

In einer bevorzugten Ausführungsform weist die Vorrichtung zur Verbindung eines ersten geschlossenen Systems mit einem Sterilkonnektor keine Hülle um zumindest Teile eines ersten und/oder zweiten geschlossenen Systems auf.

In einer bevorzugten Ausführungsform weist die Vorrichtung zur Verbindung eines ersten geschlossenen Systems mit einem Sterilkonnektor eine Halterung für ein weiteres geschlossenes System auf, welches mittels einer weiteren Kanüle oder eines weiteren Kanals mit dem Sterilkonnektor verbunden werden kann.

Die Erfindung betrifft auch ein Kit umfassend mindestens einen erfindungsgemäßen Sterilkonnektor und eine erfindungsgemäße Vorrichtung zur Verbindung eines ersten geschlossenen Systems mit einem Sterilkonnektor.

In einer bevorzugten Ausführungsform umfasst das Kit ferner mindestens ein erstes geschlossenes System.

Die vorliegende Erfindung betrifft auch die Verwendung einer Vorrichtung zur Verbindung eines ersten geschlossenen Systems mit einem Sterilkonnektor zur sterilen Verbindung eines ersten geschlossenen Systems mit einem, insbesondere erfindungsgemäßen, Sterilkonnektor.

In einer bevorzugten Ausführungsform ist eine erfindungsgemäße Verwendung eine Verwendung im klinischen Alltag, bei einem Rettungseinsatz, in einer ärztlichen Praxis, insbesondere beim Anlegen einer Infusionslösung. Dies hat den Vorteil, dass das Immunsystem eines menschlichen oder tierischen Patienten entlastet wird.

Im Zusammenhang mit der vorliegenden Erfindung wird unter dem Begriff "steriler Verbindung" eine Verbindung verstanden, welche zwischen zwei oder mehreren geschlossenen Systemen hergestellt wird und welche dabei die Geschlossenheit dieser Systeme nicht oder nur unwesentlich ändert, daher die Systeme haben weiterhin keinen Kontakt mit der Umgebung, insbesondere der unsterilen Umgebung, außer der Verbindung zwischen den geschlossenen Systemen selbst. Ein oder beide geschlossene Systeme können in einer besonderen Ausführungsform steril sein.

Im Zusammenhang mit der vorliegenden Erfindung wird unter dem Begriff "erhitzungsresistent" eine Eigenschaft eines Materials verstanden, welches sich dadurch auszeichnet, dass es erst ab einer höheren Temperatur, insbesondere ab 180 °C, seinen Aggregatzustand ändert von fest zu flüssig oder seine molekulare Struktur zerstört wird.

Im Zusammenhang mit der vorliegenden Erfindung wird unter dem Begriff "geschlossenes System" ein System verstanden, welches ausschließlich gasförmige, insbesondere keine, Materie mit seiner Umgebung austauscht.

Im Zusammenhang mit der vorliegenden Erfindung wird unter dem Begriff "Fluid-Verbindung" eine Verbindung verstanden, welche es erlaubt Flüssigkeiten oder Gase zu transportieren, insbesondere von einem System in ein anderes.

Im Zusammenhang mit der vorliegenden Erfindung wird unter dem Begriff "fluiddicht" ein Zustand eines Systems oder Verbindung verstanden, bei welchem zwischen zwei Bereichen, insbesondere dem Inneren eines Systems oder einer Verbindung und der Umgebung, nur ein unwesentlicher oder kein Austausch von flüssiger oder fester Materie, insbesondere auch keiner gasförmigen Materie, stattfinden kann.

Im Zusammenhang mit der vorliegenden Erfindung wird unter dem Begriff "mindestens eine" eine Mengenangabe verstanden, die eine Anzahl von 1 oder 2 oder 3 oder 4 oder 5 oder 6 oder 7 oder 8 oder 9 oder 10 und so weiter ausdrückt. In einer besonders bevorzugten Ausführungsform kann die Bezeichnung "mindestens eine" genau die Anzahl 1 darstellen. In einer weiteren bevorzugten Ausführungsform kann die Begrifflichkeit "mindestens eine" auch 2 oder 3 oder 4 oder 5 oder 6 oder 7 bedeuten.

Sofern im Zusammenhang mit der vorliegenden Erfindung quantitative Angaben, insbesondere Prozentangaben, von Komponenten eines Produktes oder einer Zusammensetzung angegeben sind, addieren diese, sofern nicht explizit anders angegeben oder fachmännisch ersichtlich, zusammen mit den anderen explizit angegeben oder fachmännisch ersichtlichen weiteren Komponenten der Zusammensetzung oder des Produktes auf 100 % der Zusammensetzung und/oder des Produktes auf.

Sofern im Zusammenhang mit der vorliegenden Erfindung ein "Vorhandensein", ein "Enthalten", ein "Aufweisen" oder ein "Gehalt" einer Komponente ausdrücklich erwähnt oder impliziert wird bedeutet dies, dass die jeweilige Komponente vorhanden ist, insbesondere in messbarer Menge vorhanden ist.

Sofern im Zusammenhang mit der vorliegenden Erfindung ein "Vorhandensein", ein "Enthalten" oder ein "Aufweisen" einer Komponente in einer Menge von 0 [Einheit], insbesondere mg/kg, µg/kg oder Gew.-%, ausdrücklich erwähnt oder impliziert wird, bedeutet dies, dass die jeweiligen Komponenten nicht in messbarer Menge vorhanden, insbesondere nicht vorhanden ist.

Die Zahl der angegebenen Nachkommastellen entspricht der Präzision der jeweils angewandten Messmethode.

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: a) A oder B oder C oder b) (A und B), oder c) (A und C), oder d) (B und C), oder e) (A und B und C).

Im Zusammenhang mit der vorliegenden Erfindung wird unter den Begriffen "umfassend" und "aufweisend" verstanden, dass zusätzlich zu den von diesen Begriffen explizit erfassten Elementen noch weitere, nicht explizit genannte Elemente hinzutreten können. Im Zusammenhang mit der vorliegenden Erfindung wird unter diesen Begriffen auch verstanden, dass allein die explizit genannten Elemente erfasst werden und keine weiteren Elemente vorliegen. In dieser besonderen Ausführungsform ist die Bedeutung der Begriffe "umfassend" und "aufweisend" gleichbedeutend mit dem Begriff "bestehend aus". Darüber hinaus erfassen die Begriffe "umfassend" und "aufweisend" auch Zusammensetzungen, die neben den explizit genannten Elementen auch weitere nicht genannte Elemente enthalten, die jedoch von funktioneller und qualitativ untergeordneter Natur sind. In dieser Ausführungsform sind die Begriffe "umfassend" und "aufweisend" gleichbedeutend mit dem Begriff "im Wesentlichen bestehend aus". Der Begriff "bestehend aus" bedeutet, dass allein die explizit genannten Elemente vorliegen und die Anwesenheit weiterer Elemente ausgeschlossen ist.

Weitere Ausführungsformen der vorliegenden Erfindung sind die Gegenstände der Unteransprüche und weiteren unabhängigen Ansprüche.

Die Erfindung wird anhand des folgenden Beispiels und der dazugehörigen Figuren näher erläutert.

Die Figuren zeigen:
Figur 1 zeigt einen Teil eines erfindungsgemäßen Sterilkonnektors in seitlicher Ansicht.
Figur 2 zeigt einen erfindungsgemäßen Sterilkonnektor in seitlicher Ansicht.
Figur 3 zeigt einen Teil eines erfindungsgemäßen Sterilkonnektors mit einer Kanüle mit zwei Kanälen, wobei der zweite Kanal an einen Sterilfilter verbunden ist, in seitlicher Ansicht als Schnittdarstellung.
Figur 4 zeigt einen erfindungsgemäßen Sterilkonnektor mit einer Kanüle mit zwei Kanälen, wobei der zweite Kanal an einen Sterilfilter verbunden ist, in seitlicher Ansicht.
Figur 5 zeigt eine erfindungsgemäße Vorrichtung zur Verbindung eines ersten geschlossenen Systems mit einem Sterilkonnektor, wobei ein Sterilkonnektor mit einem ersten geschlossenen System verbunden ist.
Figur 6 zeigt eine erfindungsgemäße Vorrichtung zur Verbindung eines ersten geschlossenen Systems mit einem Sterilkonnektor.
Figur 7 zeigt eine erfindungsgemäße Vorrichtung zur Verbindung eines ersten geschlossenen Systems mit einem Sterilkonnektor, wobei ein erstes geschlossenes System in der Befestigung für ein erstes geschlossenes System angebracht ist.
Figur 8 zeigt eine erfindungsgemäße Vorrichtung zur Verbindung eines ersten geschlossenen Systems mit einem Sterilkonnektor, wobei ein erstes geschlossenes System in der Befestigung für ein erstes geschlossenes System angebracht ist, und ein Sterilkonnektor an die kraftgetriebene mechanische Führung fixiert ist.
Figur 9 zeigt einen Teil eines erfindungsgemäßen Sterilkonnektors mit einem Teil der Vorrichtung zur Verbindung eines ersten geschlossenen Systems mit einem Sterilkonnektor und ein erstes geschlossenes System.
Figur 10 zeigt einen Teil eines erfindungsgemäßen Sterilkonnektors mit einem Teil der Vorrichtung zur Verbindung eines ersten geschlossenen Systems mit einem Sterilkonnektor und ein erstes geschlossenes System.
Figur 11 zeigt einen Teil eines erfindungsgemäßen Sterilkonnektors mit einem Teil der Vorrichtung zur Verbindung eines ersten geschlossenen Systems mit einem Sterilkonnektor und ein erstes geschlossenes System, wobei der Sterilkonnektor in Kontakt mit dem ersten geschlossenen System steht.
Figur 12 zeigt einen erfindungsgemäßen Sterilkonnektor mit einem Teil der Vorrichtung zur Verbindung eines ersten geschlossenen Systems mit einem Sterilkonnektor und einem ersten geschlossenen System.
Figur 13 zeigt einen erfindungsgemäßen Sterilkonnektor mit einem Teil der Vorrichtung zur Verbindung eines ersten geschlossenen Systems mit einem Sterilkonnektor und ein erstes geschlossenes System, wobei der Sterilkonnektor in Kontakt mit dem ersten geschlossenen System steht.
Figur 14 zeigt einen erfindungsgemäßen Sterilkonnektor fixiert an einem Teil der Vorrichtung zur Verbindung eines ersten geschlossenen Systems mit einem Sterilkonnektor und ein erstes geschlossenes System.
Figur 15 zeigt einen erfindungsgemäßen Sterilkonnektor fixiert an einem Teil der Vorrichtung zur Verbindung eines ersten geschlossenen Systems mit einem Sterilkonnektor und ein erstes geschlossenes System.
Figur 16 zeigt einen erfindungsgemäßen Sterilkonnektor fixiert an einem Teil der Vorrichtung zur Verbindung eines ersten geschlossenen Systems mit einem Sterilkonnektor und ein erstes geschlossenes System, wobei der Sterilkonnektor in Kontakt mit dem ersten geschlossenen System steht.
Figur 17 zeigt einen erfindungsgemäßen Sterilkonnektor fixiert an einem Teil der Vorrichtung zur Verbindung eines ersten geschlossenen Systems mit einem Sterilkonnektor und ein erstes geschlossenes System.
Figur 18 zeigt eine schematische Darstellung eines erfindungsgemäßen Sterilkonnektors mit einem Teil der Vorrichtung zur Verbindung eines ersten geschlossenen Systems mit einem Sterilkonnektor und ein erstes geschlossenes System.
Figur 19 zeigt eine schematische Darstellung eines erfindungsgemäßen Sterilkonnektors mit einem Teil der Vorrichtung zur Verbindung eines ersten geschlossenen Systems mit einem Sterilkonnektor und ein erstes geschlossenes System, wobei der Sterilkonnektor in Kontakt mit dem ersten geschlossenen System steht.
Figur 20 zeigt eine schematische Darstellung eines erfindungsgemäßen Sterilkonnektors mit einem Teil der Vorrichtung zur Verbindung eines ersten geschlossenen Systems mit einem Sterilkonnektor und ein erstes geschlossenes System, wobei der Sterilkonnektor mit dem ersten geschlossenen System verbunden ist.

### BEZUGSZEICHENLISTE

- 1: Sterilkonnektor
- 11: Kanüle
- 111: Einstichbereich
- 112: Anschlussbereich
- 12: Hülle
- 13: Anschlusselement
- 14: Verbindungselement
- 15: Arretiervorrichtung
- 151: Rahmenteil
- 152: Rastvorrichtung
- 153: Anschlussöffnung
- 154: Gasöffnung
- 16: Erster Kanal
- 17: Zweiter Kanal
- 18: Sterilfilter
- 2: erstes geschlossenes System
- 21: Septum (erstes geschlossenes System)
- 3: zweites geschlossenes System
- 31: Septum (zweites geschlossenes System)
- 4: Vorrichtung zur Verbindung eines ersten geschlossenen Systems mit einem zweiten geschlossenen System
- 41: Befestigung für ein erstes geschlossenes System
- 42: kraftgetriebene mechanische Führung
- 43: Vorrichtung zum Erhitzen der mindestens einen Kanüle des Sterilkonnektors
- 431: Induktionsspule als Vorrichtung zum Erhitzen der mindestens einen Kanüle des Sterilkonnektors
- 432: Induktionsheizung
- 44: beweglicher Schlitten
- 47: Fixierung Sterilkonnektor/Klammer

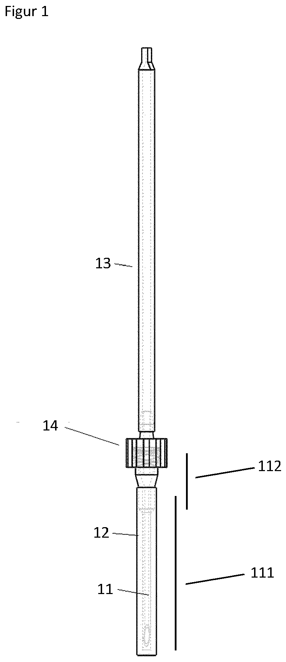

In Figur 1 wird, zur besseren Darstellung, ein Teil eines erfindungsgemäßen Sterilkonnektors gezeigt. Dargestellt ist insbesondere eine mit einer elastischen, erhitzungsresistenten Hülle 12 ummantelte Kanüle 11, wobei die Hülle den Einstichbereich 111, insbesondere auch das, vom Verbindungselement 14 aus gesehen, distale Ende und die Öffnung der Kanüle, sowie einen Teil des Anschlussbereichs 112 der Kanüle ummantelt. Die Kanüle 11 ist über das Verbindungselement 14 mit dem Anschlusselement 13 fluiddicht verbunden. Nicht dargestellt ist die Arretiervorrichtung 15.

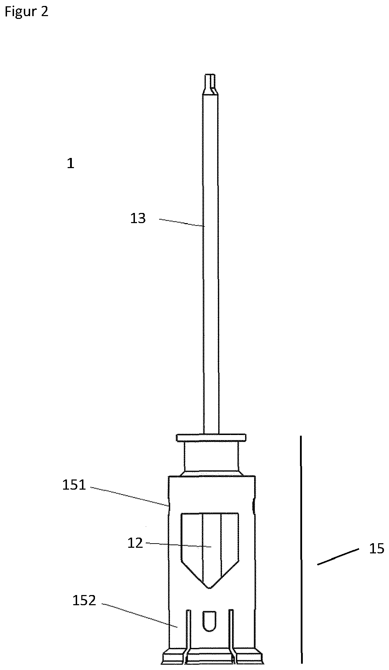

In Figur 2 wird ein erfindungsgemäßer Sterilkonnektor 1 gezeigt, wobei die Kanüle 11 wegen der Ummantelung mit der Hülle 12 und das Verbindungselement 14 aufgrund der Arretiervorrichtung 15 nicht sichtbar sind. Die gezeigte Arretiervorrichtung 15 umfasst ein Rahmenteil 151 mit Rastvorrichtungen 152. Diese Rastvorrichtungen 152 sind dazu geeignet, den Sterilkonnektor 1 an ein ersten geschlossenen System 2 zu arretieren, wobei die gezeigte Arretiervorrichtung für die Arretierung insbesondere an eine Bördelflasche oder geschlossene System mit ähnlichen Anschlüssen ausgelegt ist. Ebenso gezeigt ist, dass die Arretiervorrichtung 15 die Kanüle 11 allseitig unter Bildung einer Anschlussöffnung 153(nicht gezeigt) umfasst, wobei die Hülle 12 durch die Arretiervorrichtung 15 nicht kontaktiert wird.

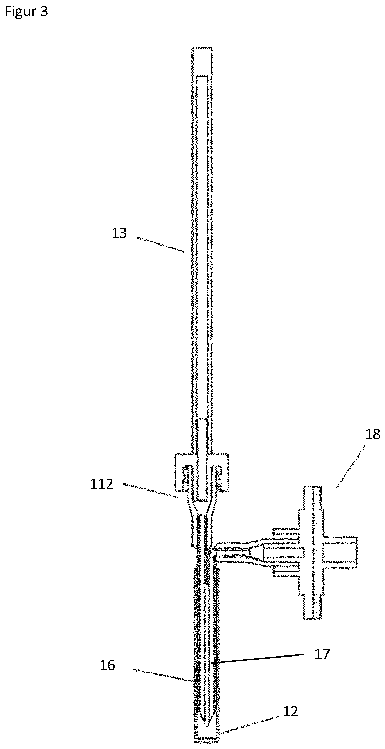

Figur 3 zeigt einen Teil einer alternativen Ausführungsform eines erfindungsgemäßen Sterilkonnektors, wobei diese Ausführungsform eine Kanüle 11 aufweist, welche zwei Kanäle 16, 17 aufweist, im Querschnitt. Der erste Kanal 16 steht in Fluidverbindung mit dem Anschlusselement über den Anschlussbereich 112 und dem Verbindungselement 14. Der zweite Kanal 17 weist einen separaten Anschlussbereich auf, wobei über diesen Anschlussbereich der zweite Kanal 17 mit der Umgebung über einen Sterilfilter 18 in Verbindung steht. Ebenso ist in dieser Ausführungsform die Kanüle 11 mit der Hülle 12 ummantelt.

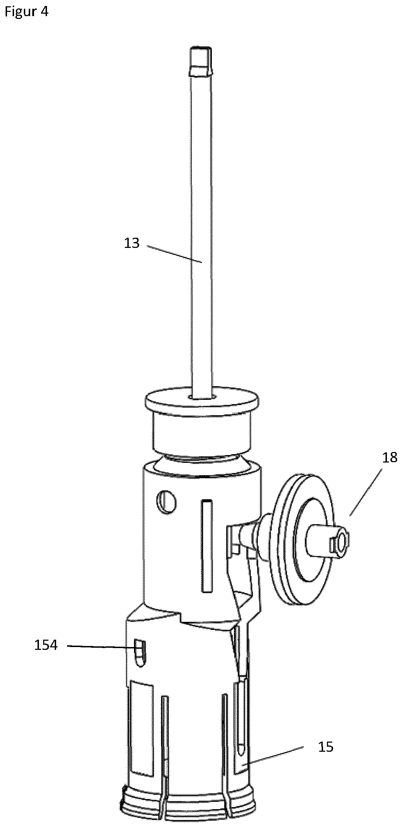

Auch diese Ausführungsform mit zwei Kanälen weist eine Arretiervorrichtung 15 auf (Figur 4). Diese Ausführungsform weist ebenso seitliche Öffnungen/Gasöffnungen 154 auf, welche es ermöglichen einen Gasstrom auf die Hülle um die Kanüle und/oder dem Verbindungselement zu richten, um diese bei einer Erhitzung der Kanüle zu kühlen.

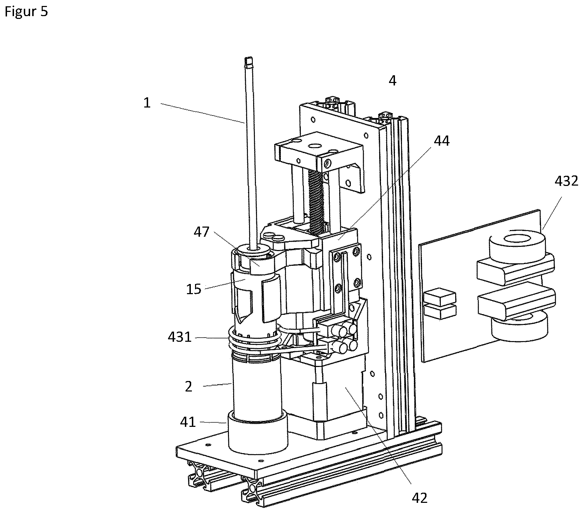

In Figur 5 ist eine Vorrichtung zur Verbindung eines ersten geschlossenen Systems mit einem Sterilkonnektor 4 gezeigt. In dieser Darstellung ist ebenso ein Sterilkonnektor 1 an der kraftgetriebenen mechanischen Führung 42 über einen Schlitten 44 und eine Klammer 47 reversibel fixiert. Die mechanische Führung 42 bewegt dabei den Schlitten 44 und somit auch den Sterilkonnektor 1. Die Vorrichtung 4 weist ferner eine Induktionsspule 431 auf, welche über eine Induktionsheizung 432 mit Energie versorgt wird, um die Kanüle 11 zu erhitzen. Die Induktionsspule 431 ist dabei an dem Schlitten 44 befestigt und um die Arretiervorrichtung 15 und somit auch um die Kanüle 11 angeordnet, um diese, insbesondere während der Schlitten 44 bewegt wird, zu erhitzen. Ferner weist die dargestellte Vorrichtung 4 eine Befestigung für ein erstes geschlossenes System 41 auf, wobei in der dargestellten Ausführungsform die Befestigung ausgelegt ist, eine Bördelflasche als erstes geschlossenes System 2 aufzunehmen. Das erste geschlossene System 2 ist dabei vorzugsweise so fixiert, dass dieses nicht durch die entstehende Kraft der eindringenden Kanüle und der Arretiervorrichtung bewegt wird. In der dargestellten Ausführungsform ist der Sterilkonnektor 1 mit dem ersten geschlossenen System 2 durch die Vorrichtung 4 verbunden.

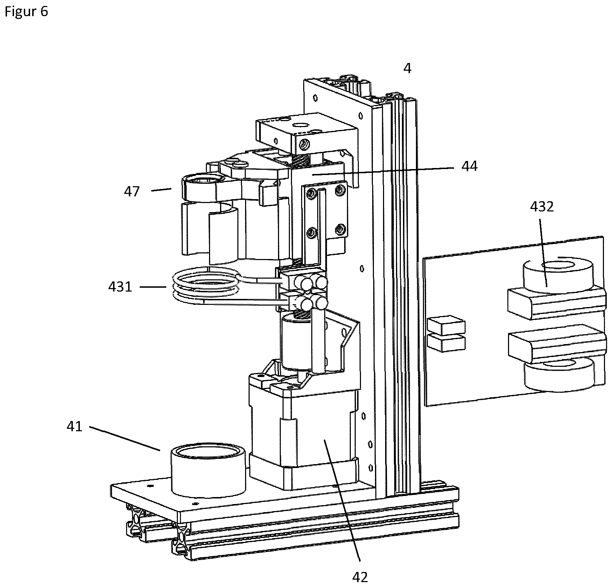

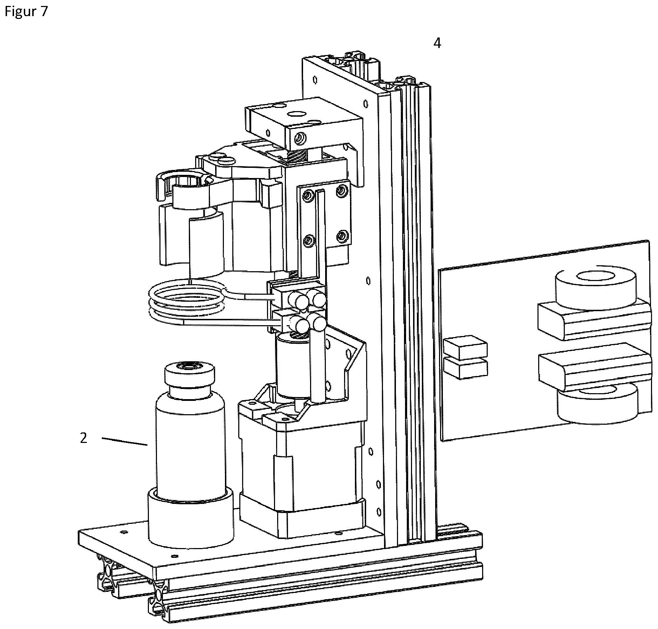

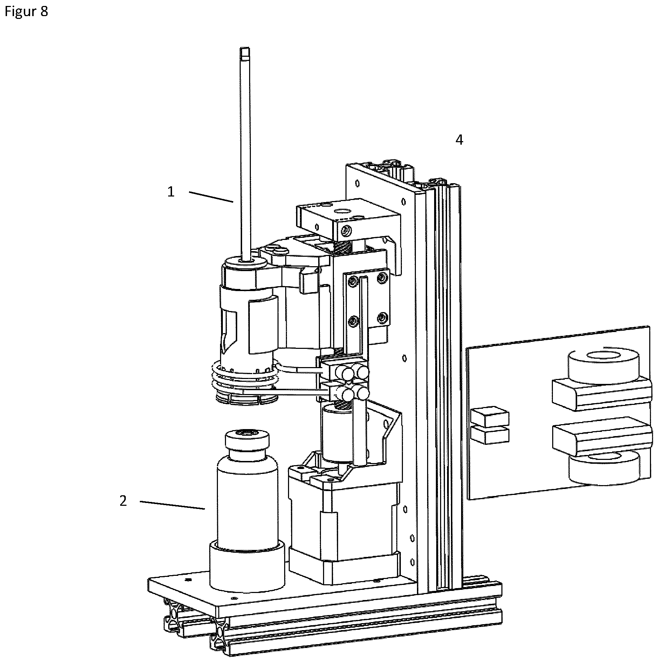

In Figur 6 ist die gleiche Vorrichtung 4 wie in Figur 5 ohne Sterilkonnektor 1 oder erstem geschlossenen System 2 dargestellt. Der bewegliche Schlitten 44 der mechanischen Führung 42 ist in Figur 6 in einer Position, in welcher der Sterilkonnektor und das erste geschlossene System vor dem Verbinden an der Vorrichtung 4 befestigt werden können. Zur Befestigung des ersten geschlossenen Systems kann dieses in die Befestigung 41 eingeführt werden (Figur 7). Anschließend kann ebenso der Sterilkonnektor 1 an den Schlitten 44 mit der Klammer 47 fixiert werden (Figur 8). Alternativ kann ebenso erst der Sterilkonnektor 1 und anschließend das erste geschlossene System 2 befestigt werden.

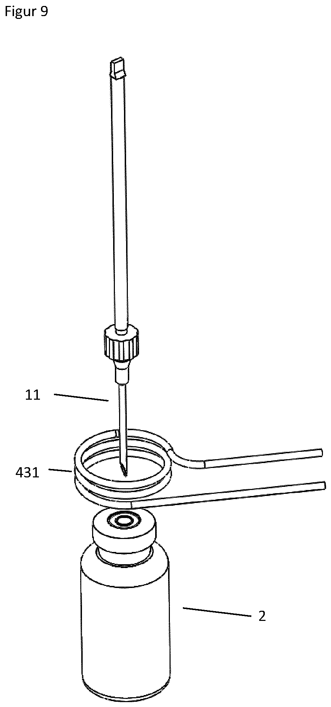

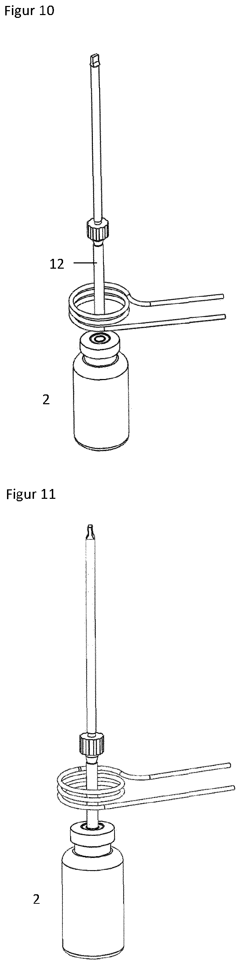

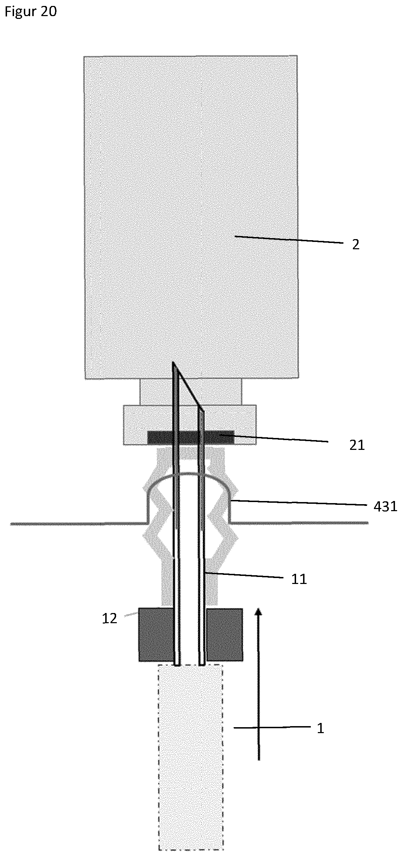

In Figur 9 ist der Sterilkonnektor 1 ohne Hülle 12 (in Figur 10 mit Hülle) und ohne Arretiervorrichtung 15 im Verhältnis zur Induktionsspule 431 der Vorrichtung 4 und einer Bördelflasche als erstem geschlossene System dargestellt, wobei der Sterilkonnektor nicht mit der Bördelflasche verbunden ist. In Figur 11 ist das Verhältnis dieser zueinander dargestellt, wenn der Sterilkonnektor 1 das erste geschlossene System 2 kontaktiert (siehe auch Figur 20).

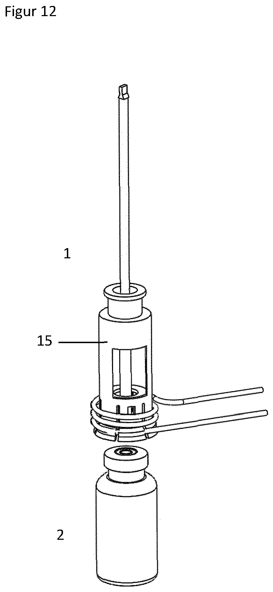

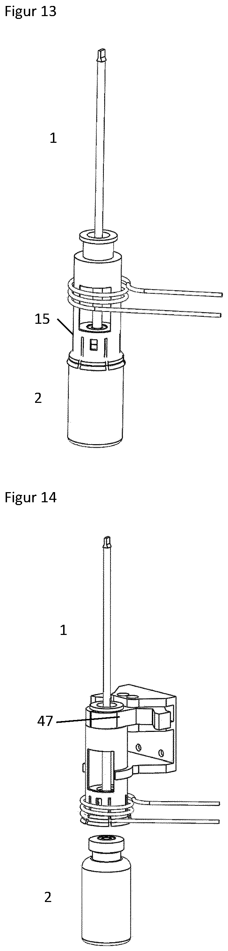

In Figuren 12 und 13 ist der vollständige erfindungsgemäße Sterilkonnektor 1 mit einem Teil der Vorrichtung 4 sowie einer Bördelflasche als erstem geschlossene System dargestellt. In Figur 12 sind der Sterilkonnektor 1 und das erste geschlossene System nicht verbunden, wohingegen in Figur 13 diese Verbindung hergestellt ist. Hierbei ist insbesondere auch dargestellt, wie das erste geschlossene System über die Anschlussöffnung 153 in den ersten Sterilkonnektor aufgenommen wird. Ebenso ist sichtbar, dass die Rastvorrichtung 152 so eingerastet ist, dass das erste geschlossene System 2 arretiert wird.

Figur 14 zeigt die gleiche Darstellung wie Figuren 9, 10 und 12, jedoch wird zusätzlich der Schlitten 44 der Vorrichtung 4 gezeigt sowie eine Klammer als Fixierung 47 des Sterilkonnektors 1.

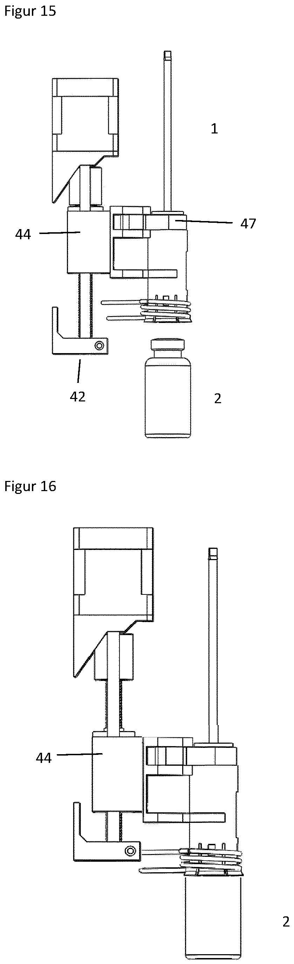

Figur 15 zeigt zusätzlich zur Figur 14 eine mechanische Führung 42, auf der der Schlitten 44 bewegt werden kann, um den Sterilkonnektor 1 mit dem ersten geschlossenen System 2 zu verbinden. In Figur 16 ist die Verbindung zwischen Sterilkonnektor 1 und erstem geschlossenen System 2 analog zu der Darstellung in der Figur 13 gezeigt. In Figuren 15 und 16 ist erkennbar, dass Ausführungen der Vorrichtung 4 möglich sind, in welcher die Induktionsspule 431 nicht auf dem Schlitten 44 montiert ist.

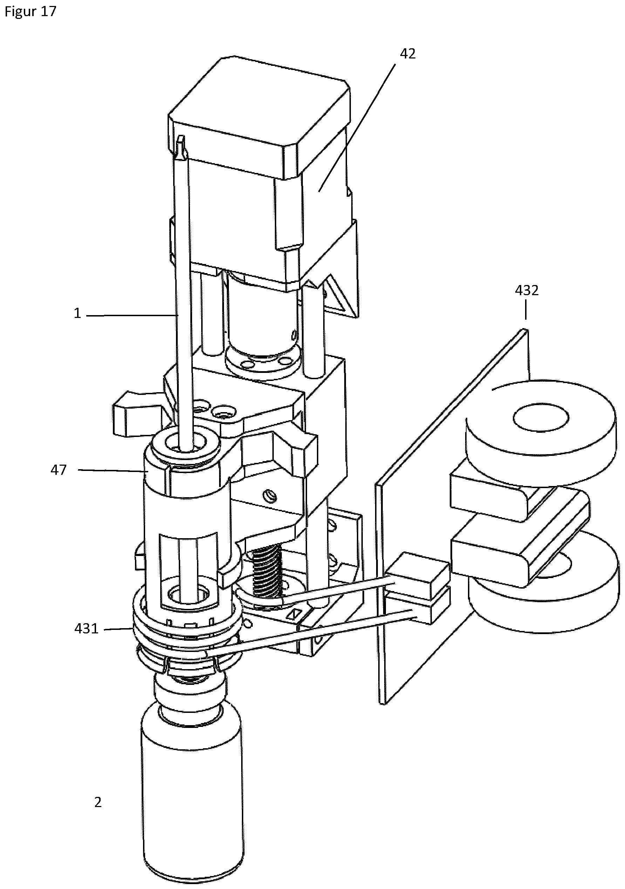

In Figur 17 sind in einer anderen Perspektive Teile einer Vorrichtung 4 sowie ein Sterilkonnektor 1, welcher an dieser Vorrichtung fixiert ist, sowie ein erstes geschlossenes System 2 dargestellt. In dieser Ausführungsform ist eine Induktionsspule 431 mit Induktionsheizung 432 dargestellt, wobei die Induktionsspule nicht auf dem Schlitten 44 montiert ist.

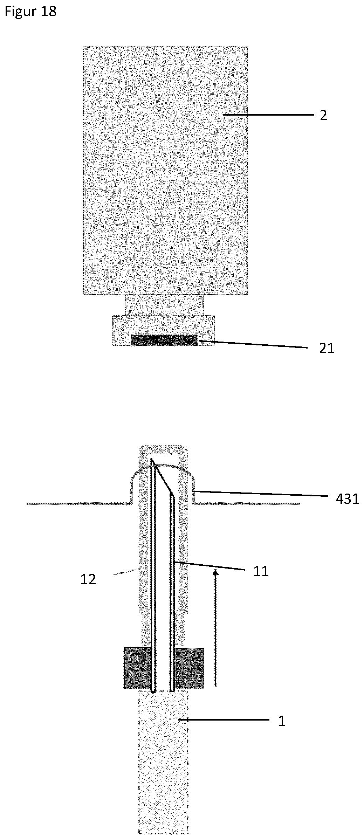

Der Ablauf der Verbindung zwischen Sterilkonnektor 1 und erstem geschlossenen System 2 wird schematisch in den Figuren 18 bis 20 dargestellt. Zunächst werden das erste geschlossene System 2 und der Sterilkonnektor 1 bereitgestellt, indem sie in räumliche Nähe zueinander gebracht werden, wobei das distale Ende der Kanüle (gesehen vom Verbindungselement) auf das Septum 21 des ersten geschlossenen Systems ausgerichtet ist. Hierbei wird der Sterilkonnektor 1 ferner so positioniert, dass eine Induktionsspule 431 die Kanüle 11 aus induktiv erhitzbarem Material erhitzen kann, indem Energie per Induktion übertragen wird (Figur 18). Die Bewegung des Sterilkonnektors 1 zum ersten geschlossenen System 2 wird durch den Pfeil neben dem Konnektor in den Figuren 18 bis 20 veranschaulicht.

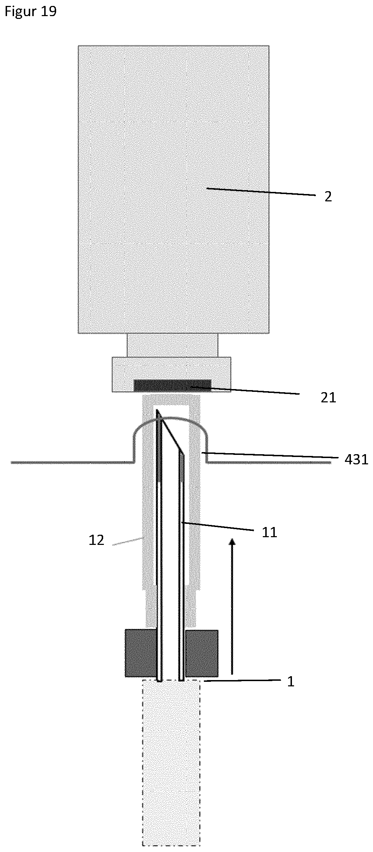

Bevor der Sterilkonnektor 1 mit dem ersten geschlossenen System 2 verbunden wird, wird die Kanüle mittels Induktion erhitzt (Figur 19, dunkler Bereich der Kanüle 11), wobei hier sowohl der Sterilkonnektor als auch die Induktionsspule schon zum ersten geschlossenen System hin verfahren werden können. Ebenso kann, während die Kanüle 11 über das Septum 21 und durch die Hülle 12 in das erste geschlossene System 2 eindringt und somit eine Fluid-Verbindung herstellt, die Kanüle 11 über die Induktionsspule 431 weiter erhitzt werden (Figur 20). Dadurch, dass die Hülle und das Septum durch die Kanüle erst zu einem Zeitpunkt durchstochen werden, bei welchen die Kanüle kurz zuvor noch erhitzt wurde oder noch wird, können Kontaminationen, welche sich auf der Hülle 12 oder dem Septum 21 befinden, durch die Hitze abgetötet werden und dringen nicht in einem vermehrungsfähigen Zustand in das erste geschlossene System 2 oder den Sterilkonnektor 1 ein. Während die Kanüle 11 des Sterilkonnektors 1 in das erste geschlossene System 2 eindringt, bleibt die Hülle 12, welche die Kanüle ummantelt, außerhalb des ersten geschlossenen Systems.

### DETAILLIERTE BESCHREIBUNG BEVORZUGTER AUSFÜHRUNGSFORMEN:

A) Ein Gefäß, als erstes geschlossenes System 2, wird mit einem zweiten geschlossenen System 3 mittels eines erfindungsgemäßen Sterilkonnektors 1 steril verbunden. Die Sterilität wird über eine Heißsterilisierung mit Induktionshitze erzeugt.

Das Gefäß 2 verfügt über ein Septum 21 und ist bevorzugt im Inneren steril. Das Septum 21 ist aus einem Elastomer gefertigt.

Das erste und zweite geschlossene System 2, 3 ist ein, vorzugsweise steriles, Behältnis, welches eine beliebige Komplexität haben kann. Im einfachsten Fall ist das zweite geschlossene System 3 ein kurzes Stück Schlauch mit einem versiegelten Ende. Dieser Schlauch kann nach dem Verbinden dann mittels Schlauchschweißens mit weiteren Behältnissen und Anwendungen verbunden werden. In einem komplexeren Fall ist es zum Beispiel ein Schlauchset, in welchem vollständige Zellkultur- und Transformationsprozesse durchgeführt werden können.

Der Sterilkonnektor 1 umfasst eine induktiv erhitzbare Kanüle 11, welche von einer flexiblen Hülle 12 ummantelt ist und mit einem Verbindungselement 14 und einer Arretiervorrichtung 15 verbunden ist. Das Verbindungselement 14 ist zusätzlich mit dem Anschlusselement 13 verbunden. Der Sterilkonnektor 1 ist, wie vorzugsweise das Gefäß und das zweite geschlossene System, im Inneren steril. Die Kanüle 11 wird induktiv erhitzt und in das Gefäß 2 eingestochen, wobei die flexible Hülle 12, die auf dem Septum 21 des Gefäßes 2 aufsetzt, zuerst durchstochen wird.

Die Kanüle 11 kann in verschiedenen Ausführungsformen mit einem einzelnen Kanal oder mit zwei beziehungsweise mehreren Kanälen 16,17 vorliegen. Ein Kanal 16 bildet die Verbindung zum zweiten geschlossenen System 3 aus. Ein zweiter Kanal 17 kann dazu genutzt werden einen bei der Entnahme der Flüssigkeit entstehenden Unterdruck oder Überdruck auszugleichen. Hierzu ist am Ende des zweiten Kanals 17 ein Sterilfilter 18 oder ein verformbarer Behälter mit steriler Luft oder einem oder mehreren Schutzgasen angebracht. Wenn die Kanüle 11 in das Gefäß 2 eingestochen ist, dichtet das elastische Septum 21 die Verbindung an der Kanüle 11 ab. Der Anpressdruck bestimmt hierbei die Druckdichtigkeit, die die Verbindung aushält. Wenn zum Beispiel, insbesondere sterile, Luft über das angeschlossene System in das Gefäß 2 eingebracht wird, wird die Verbindung durch Überdruck belastet. Beim Überschreiten des Maximaldrucks der Verbindung am Septum 21 wird das System undicht und Luft beziehungsweise Flüssigkeit tritt aus. Im umgekehrten Fall, wenn über das angeschlossene System der Inhalt durch Unterdruck entnommen wird entsteht ein gleichgroßer Unterdruck im verbundenen starren Gefäß 2. Wenn auf diese Weise die Druckdifferenz zwischen der (im Wesentlichen atmosphärischen) Umgebung und dem Inneren des Gefäßes 2 einen Wert überschreitet, wird Luft durch einen entstehenden Spalt zwischen Septum 21 und Kanüle 11 eintreten. Diese Luft ist im allgemeinen insteril, außer der Vorgang findet in einer sterilen Umgebung statt. Das kann eine sterile Werkbank oder ein steriler Raum (A-Bereich von Reinräumen gemäß EU-GMP-Leitfaden gemäß § 2 Nr. 3 der Arzneimittel- und Wirkstoffherstellungsverordnung der Bundesrepublik Deutschland) sein.

Der Eintritt von in steriler Luft beziehungsweise der Austritt von Flüssigkeiten oder Gasen wird in der Anwendung des erfindungsgemäßen Sterilkonnektors 1 vermieden. Das System ist daher in einer Ausführungsform mit nur einem Kanülen-Kanal 16 vorzugsweise nur bis zu einem gewissen Druckwert zu betreiben. Es kann vor der Entleerung ein steriler "Luftvorrat" in das Gefäß 2 eingebracht werden, der einen unkritischen Überdruck erzeugt. Daraufhin kann Flüssigkeit entnommen werden bis zu einem unkritischen Unterdruck. Dieser Vorgang lässt sich wiederholen, insbesondere solange innerhalb der Druckgrenzen des Systems gearbeitet wird und innerhalb des angeschlossenen Systems der Luftstrom und die Flüssigkeit gesteuert wird. Im einfachsten Fall wäre dies zum Beispiel eine am Anschlusselement 13 verbundene Spritze, welche vorab mit Luft aufgezogen ist.

In einer Ausführungsform des Sterilkonnektors 1 mit einem oder mehreren zusätzlichen Kanälen kann die Druckdifferenz durch einen Sterilfilter 18 oder ein steriles Luftreservoir ausgeglichen werden und so das Gefäß entleert oder befüllt werden, ohne dass relevante Druckdifferenzen entstehen.

Mit den beschriebenen Vorgehensweisen kann das System daher auch außerhalb steriler Räume Anwendung finden.

### B) Induktionsspule als Hufeisen oder mit Öffnungsmechanismus

Ein wechselndes Magnetfeld im Bereich 50-800 kHz wird mittels einer Induktionsspule 431 um die induktiv erhitzbare Kanüle 11 erzeugt. Die Induktionsspule 431 kann hierbei die Kanüle 11 als Spirale umschließen oder offen in Form eines "Hufeisen" ummanteln. Letzteres hat den Vorteil, dass ein mit dem erfindungsgemäßen Sterilkonnektor verbundenes Gefäß leichter entnommen werden kann. Bevorzugt ist auch eine Induktionsspule 431, welche mechanisch klappbar gebaut ist und vor und nach dem Prozess geöffnet werden kann.

Das Magnetfeld wird im unteren Bereich der Kanüle 11 erzeugt und erhitzt diese in wenigen Sekunden auf eine Temperatur von 60-300°C, welche vorhandene Keime schnell zerstört. Die Wärme verteilt sich in der Kanüle 11 mit einer gewissen Verzögerung, so dass der verbundene Kunststoff des Sterilkonnektors nicht geschmolzen wird. Die elastische Hülle 12 kann nicht schmelzen.

### C) Sterilkonnektor- Kanülenhülle mit Arretiervorrichtung

Der erfindungsgemäße Sterilkonnektor 1 umfasst eine induktiv erhitzbare Kanüle 11, die auf der Einstichseite von einer flexiblen Hülle 12 ummantelt ist und am anderen Ende mit einem Anschlusselement 13, zum Beispiel einem versiegelten, kurzen, medizinischen PVC-Schlauch verbunden ist. An dem Verbindungselement 14 ist zudem eine Arretiervorrichtung 15 kraftschlüssig oder integral angebracht. Die Kanüle 11 wird von der Arretiervorrichtung 15 vorzugsweise in einer Weise umhüllt, dass die flexible Hülle 12 gar nicht oder nicht fixierend berührt wird. Die Anschlussöffnung 153 der Arretiervorrichtung 15 ist spezifisch für ein standardisiertes Gefäß 2 angefertigt, zum Beispiel für ein Bördelfläschchen oder ein Plastikvial. Die Arretiervorrichtung 15 hat einen Einrastmechanismus 152, welcher gleichzeitig beim Einstechen der Kanüle 11 einrastet und danach nur mit gezielter Kraftanstrengung wieder lösbar ist. Das Einrasten fixiert die Kanüle 11 auf dem Zielgefäß 2 und verhindert, dass diese sich hin- und herbewegen kann, was potentiell eine Kontamination verursachen könnte, sobald die Kanüle 11 wieder abgekühlt ist und dass die Kanüle 11 vor dem Abkühlen den Inhalt des Gefäßes 2 berührt und damit Hitzeschäden verursachen könnte.

Die erfindungsgemäße Verbindung in Form des erfindungsgemäßen Sterilkonnektors 1 ist vorzugsweise permanent und nicht dazu ausgelegt wieder steril lösbar zu sein. In einer weiteren Ausführungsform kann jedoch auch eine lösbare Verbindung bereitgestellt werden, solange gefäßseitig das Septum 21 beziehungsweise auf der Seite des Sterilkonnektors 1 die flexible Hülle 12 sich durch ihre elastische Spannung wieder selbst verschließt, nachdem die Kanüle 11 wieder nach einer erneuten induktiven Erhitzung herausgezogen wurde. Bevorzugt erfolgt das Abtrennen des verbundenen Gefäßes 2 vom zweiten geschlossenen System 3 dadurch, dass zum Beispiel der Schlauch, an dem der Sterilkonnektor 1 verbunden ist, absiegelt wird. Das Gefäß 2 mit dem verbundenen Sterilkonnektor kann danach bevorzugt wieder abgetrennt werden.

### D) Vorrichtung mit Führung zum prozesssicheren Führen des Sterilkonnektors

Zum Verbinden des Gefäßes 2 mit dem erfindungsgemäßen Sterilkonnektor 1 dient eine kraftgetriebene mechanische Führung 42 (Linearaktuator), an dessen beweglichem Teil 44 (Schlitten) der Sterilkonnektor 1 kraftschlüssig reversibel, zum Beispiel mit einer Klammer, fixiert ist. Der Sterilkonnektor 1 wird zum Verbinden in die Vorrichtung 4 eingelegt, so dass die Kanülenspitze auf das Septum 21 des Gefäßes 2 gerichtet ist. Der Sterilkonnektor 1 kann so vom Linearaktuator 42 in Richtung des Zielgefäßes 2 positions-, geschwindigkeits- oder kraftgesteuert bewegt werden. Die Induktionsspule 431 ist zusätzlich am Schlitten 44 befestigt und überdeckt mit dem entstehenden magnetischen Wechselfeld den Einstichbereich 111 der Kanüle 11. Die Induktionsspule 431 verfährt somit gleichzeitig mit der Kanüle 11 und kann während des Einstechvorgangs weiter die Kanüle 11 aufheizen, so dass eine eventuelle verfrühte Abkühlung der Kanüle 11 durch zum Beispiel die Wärmeverluste am Septum 21 vermieden wird. Die mechanische Führung 42 kann zum Beispiel mit einem Encoder/Steuereinheit die Einstechbewegung geregelt durchführen, so dass eine gleichmäßige Einstechgeschwindigkeit erreicht wird. Das Einstellen des Sterilkonnektors 1 in die Vorrichtung 4 muss im Fall einer Induktionsspule 431, die als Spirale ausgeführt ist, so erfolgen, dass der Sterilkonnektor 1 innerhalb der Spule 431 positioniert wird, was deutlich umständlicher ist, als bei einer hufeisenförmigen Induktionsspule.

Das zu verbindende Gefäß 2 wird auch an der Vorrichtung 4 eingestellt und fixiert. Dies geschieht in einer Weise, dass das Gefäß 2 nicht durch die entstehende Kraft der eindringenden Kanüle 11 und vor allem der Kraft, die von der Arretiervorrichtung 15 einwirkt, bewegt wird. Die Ausrichtung der Kanüle 11 auf das Septum 21 des Gefäßes 2 ist vorzugsweise konzentrisch. Hierbei ist die Arretiervorrichtung 15 auf die Geometrie des Gefäßes 2, die nicht in jedem Fall eine runde Form oder ein mittig ausgerichtetes Septum 21 hat, konstruktiv abgestimmt, das heißt zu dessen Arretierung ausgelegt.

Nach dem Verbinden des Sterilkonnektors 1 mit dem Gefäß 2 können diese zusammen entnommen werden. Wenn die Induktionsspule 431 als Spirale um den Sterilkonnektor 1 gewunden ist, muss entweder das Gefäß 2 oder das Anschlusselement 13 von den Dimensionen her klein genug sein, um zur Entfernung durch die Induktionsspule 431 zu passen. Die mechanische Führung 42 kann sich nach dem Verbinden wieder nach oben bewegen, so dass der verbundene Sterilkonnektor 1 entnommen werden kann. Ob sich bei diesem Prozess, der verbundene Sterilkonnektor 1 mitbewegt oder automatisch aus der Fixierung freigegeben wird, hängt von der Ausgestaltung des Mechanismus ab.

Die Vorrichtung 4 ist bevorzugt als Handgerät ausgeführt, welches während des Prozesses gehalten werden kann. In dieser Ausführung kann durch die Handhabung sichergestellt werden, dass eine Flüssigkeit im Gefäß 2 nicht in Kontakt mit der heißen Kanüle 11 gerät, was zu einer zu schnellen Abkühlung der Kanüle 11 führen könnte und zudem das Medium teilweise zerstören/denaturieren könnte, sollte es sich zum Beispiel um Proteine oder virale Vektoren handeln.

Um eine übermäßige Erhitzung der Kanüle 11 zu vermeiden, die zu einem Schmelzen der Kanülenfassung/Anschlussbereichs 14 oder anderer in der Nähe positionierter Teile des Sterilkonnektors, zum Beispiel der Arretiervorrichtung 15, führen könnte, kann ein Gasstrom zur Kühlung des oberen Bereichs der Kanüle 11, insbesondere inklusive des Verbindungselements, verwendet werden. Eine solche Luftkühlung kann zum Beispiel ein Druckluftanschluss sein, welcher zusätzlich am Schlitten 44 der mechanischen Führung 42 angebracht ist und durch den ein kühlender Gasstrom in eine dafür vorgesehene Öffnung 154 am Sterilkonnektor eingebracht werden kann.

Die Vorrichtung 4 kann, als Handgerät ausgeführt, zusammen mit dem Sterilkonnektor 1 im klinischen Alltag oder in der ärztlichen Praxis, zum Beispiel beim Rettungseinsatz beim Anlegen von Infusionslösungen verwendet werden und hilft so, das Immunsystem eines Patienten zu entlasten.

Insbesondere kann die Vorrichtung 4 zusammen mit dem Sterilkonnektor 1 auch stationär in eine Vielzahl von geschlossenen Produktionssystemen verbaut werden. Hier kommen sämtliche Bioreaktorsysteme, die vor einer Verkeimung geschützt werden müssen, in Frage.

### E) Verfahren zum Verbinden mit kontrolliertem Prozess

Das Verfahren zum Verbinden des Gefäßes 2 mit dem erfindungsgemäßen Sterilkonnektor zeichnet sich insbesondere dadurch aus, dass eine PC- oder Mikrocontroller-Steuerung als Steuereinheit den Prozess anhand der Sensorik und Aktuatorik des Systems durchführt und ein Einhalten der kritischen Prozessparameter sicherstellt. Diese Parameter sind hierbei zum Beispiel die Temperatur der Kanüle 11, die Einstechgeschwindigkeit und das Erreichen der Einrastposition des Arretiermechanismus.
- Die Temperatur ist wichtig für die Effektivität der Heißsterilisierung. Da eine zu hohe oder zu lang andauernde Erhitzung des Sterilkonnektors vermieden werden muss, sollte die eingebrachte Energie gesteuert werden.
- Das gerade, gleichmäßige Einstechen der Kanüle 11 ist ein wichtiger Prozessparameter, da eine Hin- und Herbewegung der Kanüle 11 vermieden werden sollte, sowie ein zu schnelles oder zu langsames Einstechen. Zudem darf der Einstechwinkel nicht schräg sein damit die Arretierung korrekt erfolgen kann.
- Positions-, Geschwindigkeits- oder Kraftsteuerung des Einstechprozesses kann durch die mechanische Führung erfolgen. Ein Encoder kann zum Beispiel die Position eines Schrittes oder Servomotors rückmelden, um diese als Regelgröße zu verwenden. Hierzu könnte auch eine Messung des Stroms am Motor dienen um Schrittverluste zu erkennen. Auch eine modellbasierte Regelung des aufgebrachten Motormoments könnte über den Stromfluss stattfinden.
- Eine Kraftmessdose kann die einwirkende Kraft auf das Gefäß 2 oder auf den Schlitten 44 messen und einerseits als Regelgröße einsetzen. Andererseits kann die gemessene Kraft gegen einen erwarteten Kraftverlauf verglichen und damit als Prüffunktion verwendet werden um Fehler im Prozess zu erkennen.
- Die eingekoppelte Energie der Induktionsspule 431 kann über den Stromverbrauch des Induktionsheizers 432 ermittelt werden. Hiermit lässt sich die Temperatur der Kanüle 11 indirekt steuern, wenn das Aufheizverhalten der Kanüle 11 abhängig von Material, Geometrie und Masse der Kanüle 11 bekannt ist. Bevorzugt ist also ein vorgesteuerter Betrieb des Induktionsschwingkreises, welcher eine definierte Zeit abhängig vom eingekoppelten Stromfluss heizt, beziehungsweise solange heizt, bis die Kanüle 11 eingestochen ist, aber die Eingangsspannung entsprechend der benötigten Induktionsenergie anpasst, zum Beispiel über einen gepulsten Betrieb der Schaltung oder einer Modulation der Eingangsspannung. Die Kalibrierung der Induktionsspule 431 kann am System ohne eingelegten Sterilkonnektor erfolgen. -Ein kühlender Gasstrom kann verwendet werden, um eine übermäßige Erhitzung der Kanüle 11 zu verhindern. Hierbei kann ein Gleichgewicht aus eingebrachter Induktionswärme und abgeführter Energie durch die Gasbewegung erzeugt werden und die Kanüle 11 auf diese Weise nur im unteren Einstichbereich 111 auf einer sterilisierenden Temperatur gehalten werden.

Die zuvor genannten besonderen Ausführungsformen A) bis E) können in bevorzugter Weise miteinander jeweils kombiniert werden.

### BEISPIEL:

Eine beispielhafte Verfahrensführung sieht wie folgt aus:
1. Zunächst wird der Sterilkonnektor 1 in die Vorrichtung 4 eingebracht. Dann wird das zu verbindende Vial als erstes geschlossenes System 2 in die Vorrichtung 4 unter dem Sterilkonnektor 1 platziert.
2. Durch Auslösen des Verbindungsvorgangs wird die Induktionsspule 431 eingeschaltet und erhitzt die Kanüle 11 kontaktlos durch die Hülle 12 hindurch auf über 160°C.
3. Nach Erreichen der Temperatur fährt die Vorrichtung 4 den Sterilkonnektor 1 nach unten durch die Hülle 12 und den formschlüssig daran aufliegenden Stopfen/Septum 21 in das Lumen des Vials 2. Gleichzeitig arretiert die Arretiervorrichtung 15 des Sterilkonnektors 1 das Vial 2 so, dass die Kanüle 11 nicht weiter eindringen kann und eine stabile Verbindung zwischen Vial 2 und Sterilkonnektor 1 hergestellt wird.
4. Nach Abkühlen der Kanüle 11 kann das Vial 2 mit dem daran befestigten Sterilkonnektor 1 aus der Vorrichtung 4 entnommen werden und mittels Schlauchweißens am Zielgefäß als zweites geschlossenes System 3 konnektiert werden. Durch auf den Kopf stellen kann dann der Inhalt des Vials 2 zum Beispiel mittels Absaugung in das Zielgefäß 3 überführt werden.

Vergleich der automatisierten Prozesssteuerung mit einem händischen Einstechen der erhitzten Kanüle:
Ein manuelles Verbinden von Sterilkonnektor 1 und Gefäß 2 mit gesicherten Sterilitätsbedingungen ist möglich, hat aber einige Nachteile. Die ausreichende Erhitzung der Kanüle 11 müsste sichergestellt werden und die manuell aufzubringende Kraft müsste gleichmäßig aufgebracht werden, um ein zu langsames oder auch ruckartiges Einstechen beim Überwinden des Einrastpunktes der Arretierung zu vermeiden. Zudem wäre nicht gesichert, dass die Silikonhülle 12 nicht verletzt wird und danach die Kanüle 11 beim Einstechen hin- und herbewegt wird. Diesen Risiken kann man theoretisch mit strengen Durchführungsprotokollen begegnen, aber eine maschinelle Durchführung mit definierten Prozessparametern bietet die Möglichkeit diese Risiken mit geringem Prozessaufwand auszuschalten.

## Patentansprüche

1. Sterilkonnektor (1) zur sterilen Verbindung eines ersten geschlossenen Systems (2) mit einem zweiten geschlossenen System (3) umfassend:
- mindestens eine Kanüle (11) mit einem Einstich- (111) und einem Anschlussbereich (112), wobei der Einstichbereich ein induktiv erhitzbares Material umfasst und mit einer elastisch verformbaren, erhitzungsresistenten Hülle (12) aus Kunststoff ummantelt ist,
- ein fluiddicht mit dem Anschlussbereich der Kanüle verbundenes Anschlusselement (13), welches ausgebildet ist zur Herstellung einer Fluid-Verbindung zwischen der Kanüle (11) und dem zweiten geschlossenen System (3),
- ein an der Kanüle (11) angeordnetes, die Kanüle (11) und das Anschlusselement (13) fluiddicht verbindendes Verbindungselement (14), welches ausgebildet ist zur kraftschlüssigen oder integralen Verbindung mit einer Arretiervorrichtung (15), und
- eine mit dem Verbindungselement (14) kraftschlüssig oder integral verbundene Arretiervorrichtung (15), umfassend ein Rahmenteil (151) und mindestens eine Rastvorrichtung (152), wobei die Rastvorrichtung (152) ausgebildet ist zur Arretierung des Sterilkonnektors (1) an dem ersten geschlossenen System (2) und wobei die Arretiervorrichtung (15) den ummantelten Einstichbereich (111) der Kanüle (11) allseitig unter Bildung einer Anschlussöffnung (153) für das erste geschlossene System (2) umfasst, insbesondere ohne die Hülle (12) der Kanüle zu kontaktieren.

2. Sterilkonnektor (1) nach Anspruch 1, wobei die mindestens eine Kanüle (11), insbesondere der Einstichbereich (111), zumindest teilweise aus Metall als induktiv erhitzbares Material gefertigt ist, insbesondere aus zwei Abschnitten mit unterschiedlicher Wärmekapazität.

3. Sterilkonnektor (1) nach Anspruch 1 oder 2, wobei die erhitzungsresistente Hülle (12) aus Kunststoff besteht, welcher bis zu einer Temperatur von 300 °C nicht schmilzt oder seine molekulare Struktur zerstört wird, insbesondere aus einem Elastomer, insbesondere Gummi oder Silikon.

4. Sterilkonnektor (1) nach einem der vorstehenden Ansprüche, wobei das Anschlusselement (13) ein Schlauch, Rohr, Kanal oder sonstiges zur fluiddichten Verbindung geeignetes Verbindungssystem ist, insbesondere ein Kunststoff-Schlauch, insbesondere PVC-Schlauch ist, welcher bevorzugt an dem der Kanüle abgewandten Ende verschlossen ist, ein Beutel oder ein Element beliebiger Komplexität ist.

5. Sterilkonnektor (1) nach einem der vorstehenden Ansprüche, wobei die Arretiervorrichtung (15), insbesondere die Rastvorrichtung (152), zur Arretierung an ein Gefäß oder ein Raum- oder Gefäßsystem umfassend fluiddicht und steril miteinander verbundene Bereiche, insbesondere Räume, insbesondere Gefäße, insbesondere ein System beliebiger Komplexität, ausgebildet ist.

6. Sterilkonnektor (1) nach einem der vorstehenden Ansprüche, wobei die Rastvorrichtung (152) Mittel aufweist, insbesondere solche, die einrasten, sobald die Kanüle (11) in einer gewünschten Länge in das erste geschlossene System (2) eingeführt ist, und das erste geschlossene System (2) so arretieren, dass die Kanüle (11) nicht mehr innerhalb des ersten geschlossenen Systems (2) bewegt werden kann.

7. Verfahren zum sterilen Verbinden eines ersten geschlossenen Systems (2) mit einem zweiten geschlossenen System (3) umfassend die folgenden Verfahrensschritte:
a) Bereitstellen eines ersten (2) und eines zweiten geschlossenen Systems (3), eines Sterilkonnektors (1) nach einem der Ansprüche 1 bis 6 und einer Vorrichtung zur Erhitzung der mindestens einen Kanüle des Sterilkonnektors,
b) Erhitzen mindestens eines Abschnitts der mindestens einen Kanüle (11) auf mindestens 60 °C, für eine Zeit, die ausreicht, um den mindestens einen Abschnitt zu sterilisieren,
c) Verbinden des Sterilkonnektors (1) mit dem ersten geschlossenen System durch Einführen der mindestens einen Kanüle (11) in das erste geschlossene System (2),
d) Verbinden des Sterilkonnektors (1) mit dem zweiten geschlossenen System (3) mit dem Anschlusselement (13), wodurch eine Fluid-Verbindung zwischen dem ersten (2) und zweiten geschlossenen System (3) durch den Sterilkonnektor (1) hergestellt wird,
wobei vorzugsweise Verfahrensschritte b) und c) zumindest teilweise zeitlich parallel ablaufen oder nacheinander und wobei Verfahrensschritt d) vor Verfahrensschritt b) oder nach Verfahrensschritt c) durchgeführt wird.

8. Verfahren nach Anspruch 7, wobei das erste geschlossene System (2) ein Gefäß, insbesondere eine Bördelflasche aus Glas oder Plastik, ein Zellkulturbeutel oder Blutbeutel, oder ein beliebig komplexes geschlossenes System ist und über ein Septum (21) verschlossen ist, wobei das Septum (21) vorzugsweise mindestens aus einem Elastomer besteht und wobei das Verbinden in Verfahrensschritt c) vorzugsweise durch Einstechen der mindestens einen Kanüle (11) des Sterilkonnektors (1) in das Septum (21) erfolgt.

9. Verfahren nach einem der Ansprüche 7 oder 8, wobei das zweite geschlossene System (3) einen Schlauch, insbesondere einen Kunststoffschlauch, insbesondere einen PVC-Schlauch, aufweist, über welchen in Verfahrensschritt d) der Sterilkonnektor (1) mit dem zweiten geschlossenen System (3) verbunden wird, bevorzugt mittels sterilem Schlauchschweißen.

## Claims

1. A sterile connector (1) for sterile connection of a first closed system (2) to a second closed system (3) comprising:
- at least one cannula (11) with a puncture region (111) and a junction region (112), wherein the puncture region comprises an inductively heatable material and is encased with an elastically deformable, heat-resistant sheath (12) made of plastic,
- a junction element (13) connected to the junction region of the cannula in a fluid-tight manner, which is configured to prepare a fluid connection between the cannula (11) and the second closed system (3),
- a connection element (14) arranged on the cannula (11) connecting the cannula (11) and the junction element (13) in a fluid-tight manner, which is configured for force-fit or integral connection with a locking device (15), and
- a locking device (15) connected to the connecting element (14) in a force-fit or integral manner, comprising a frame part (151) and at least one latching device (152), wherein the latching device (152) is configured to lock the sterile connector (1) to the first closed system (2) and wherein the locking device (15) comprises the encased puncture region (111) of the cannula (11) on all sides by forming a junction opening (153) for the first closed system (2), in particular without contacting the sheath (12) of the cannula.

2. The sterile connector (1) of claim 1, wherein the at least one cannula (11), in particular the puncture region (111), is at least partially made of metal as an inductively heatable material, in particular of two sections with different heat capacities.

3. The sterile connector (1) of claim 1 or 2, wherein the heat-resistant sheath (12) consists of plastic, which does not melt or its molecular structure is not destroyed up to a temperature of 300 °C, in particular of an elastomer, in particular rubber or silicone.

4. The sterile connector (1) of one of the preceding claims, wherein the junction element (13) is a tube, pipe, channel or other connection system suitable for fluid-tight connection, in particular a plastic tube, in particular a PVC tube, which is preferably closed at the end facing away from the cannula, a bag or an element of any complexity.

5. The sterile connector (1) of one of the preceding claims, wherein the locking device (15), in particular the latching device (152), is configured for locking to a vessel or a system of spaces or vessels comprising regions, in particular spaces, in particular vessels, which are connected to one another in a fluid-tight and sterile manner, in particular a system of any complexity.

6. The sterile connector (1) of one of the preceding claims, wherein the latching device (152) has means, in particular means which latch as soon as the cannula (11) is inserted into the first closed system (2) at a desired length, and lock the first closed system (2) such that the cannula (11) can no longer be moved within the first closed system (2).

7. A method for sterile connecting a first closed system (2) to a second closed system (3) comprising the following method steps:
a) providing a first (2) and a second closed system (3), a sterile connector (1) of one of claims 1 to 6 and a device for heating the at least one cannula of the sterile connector,
b) heating at least one section of the at least one cannula (11) to at least 60 °C, for a time sufficient to sterilise the at least one section,
c) connecting the sterile connector (1) to the first closed system by inserting the at least one cannula (11) into the first closed system (2),
d) connecting the sterile connector (1) to the second closed system (3) with the junction element (13), whereby a fluid connection between the first (2) and second closed system (3) is prepared by the sterile connector (1), wherein preferably method steps b) and c) run at least partially in parallel in time or one after the other and wherein method step d) is carried out before method step b) or after method step c).

8. The method of claim 7, wherein the first closed system (2) is a vessel, in particular a crimped bottle made of glass or plastic, a cell culture bag or blood bag, or a closed system of any complexity and is closed via a septum (21), wherein the septum (21) preferably consists of at least one elastomer and wherein the connecting in method step c) is preferably carried out by puncturing the at least one cannula (11) of the sterile connector (1) into the septum (21).

9. The method of one of claims 7 or 8, wherein the second closed system (3) has a tube, in particular a plastic tube, in particular a PVC tube, via which the sterile connector (1) is connected to the second closed system (3) in method step d), preferably by means of sterile tube welding.

## Revendications

1. Un connecteur stérile (1) de connexion stérile d'un premier système fermé (2) à un second système fermé (3) comprenant
- au moins une canule (11) avec une région de ponction (111) et une région de jonction (112), dans laquelle la région de ponction comprend un matériau inductivement chauffable et est encastrée avec une gaine (12) élastiquement déformable et résistante à la chaleur en plastique,
- un élément de jonction (13) connecté à la région de jonction de la canule de manière étanche aux fluides, qui est configuré pour préparer une connexion fluidique entre la canule (11) et le second système fermé (3),
- un élément de connexion (14) agencé sur la canule (11) connectant la canule (11) et l'élément de jonction (13) de manière étanche aux fluides, qui est configuré pour une connexion forcée ou intégrale avec un dispositif de verrouillage (15), et
- un dispositif de verrouillage (15) connecté à l'élément de connexion (14) de manière forcée ou intégrale, comprenant une partie de cadre (151) et au moins un dispositif de loquet (152), dans lequel le dispositif de loquet (152) est configuré pour verrouiller le connecteur stérile (1) au premier système fermé (2) et dans lequel le dispositif de verrouillage (15) comprend la région de ponction encastrée (111) de la canule (11) sur tous les côtés en formant une ouverture de jonction (153) pour le premier système fermé (2), en particulier sans entrer en contact avec la gaine (12) de la canule.

2. Le connecteur stérile (1) de la revendication 1, dans lequel la au moins une canule (11), en particulier la région de ponction (111), est au moins partiellement en métal en tant que matériau inductivement chauffable, en particulier en deux sections ayant des capacités de chaleur différentes.

3. Le connecteur stérile (1) de la revendication 1 ou 2, dans lequel la gaine résistante à la chaleur (12) est constituée en plastique, qui ne fond pas ou dont la structure moléculaire n'est pas détruite jusqu'à une température de 300 °C, en particulier d'un élastomère, en particulier du rubber ou du silicone.

4. Le connecteur stérile (1) de l'une des revendications précédentes, dans lequel l'élément de jonction (13) est un tube, un tuyau, un canal ou un autre système de connexion approprié pour une connexion étanche aux fluides, en particulier un tube en plastique, en particulier un tube en PVC, qui est préférablement fermé à l'extrémité opposée de la canule, un sac ou un élément de complexité quelconque.

5. Le connecteur stérile (1) de l'une des revendications précédentes, dans lequel le dispositif de verrouillage (15), en particulier le dispositif de loquet (152), est configuré pour se verrouiller sur un récipient ou un système d'espaces ou de récipients comprenant des régions, en particulier des espaces, en particulier des récipients, qui sont connectés les uns aux autres de manière étanche aux fluides et stérile, en particulier un système de complexité quelconque.

6. Le connecteur stérile (1) de l'une des revendications précédentes, dans lequel le dispositif de verrouillage (152) a des moyens, en particulier des moyens qui loquent aussitôt que la canule (11) est insérée dans le premier système fermé (2) à une longueur désirée, et verrouillent le premier système fermé (2) de sorte que la canule (11) ne peut plus être déplacée à l'intérieur du premier système fermé (2).

7. Un procédé pour connecter stérilement un premier système fermé (2) à un second système fermé (3) comprenant les étapes du procédé suivantes :
a) fournir un premier (2) et un second système fermé (3), un connecteur stérile (1) de l'une des revendications 1 à 6 et un dispositif de chauffage d'au moins une canule du connecteur stérile,
b) chauffer au moins une section de l'au moins une canule (11) à au moins 60 °C, pendant une durée suffisante pour stériliser l'au moins une section,
c) connecter le connecteur stérile (1) au premier système fermé en insérant au moins une canule (11) dans le premier système fermé (2),
d) connecter le connecteur stérile (1) au second système fermé (3) avec l'élément de jonction (13), de sorte qu'une connexion fluidique entre le premier (2) et le second système fermé (3) est préparée par le connecteur stérile (1), dans lequel, préférablement, les procédés b) et c) se déroulent au moins partiellement en parallèle dans le temps ou l'un après l'autre et dans lequel le procédé d) est effectué avant le procédé b) ou après le procédé c).

8. Le procédé de la revendication 7, dans lequel le premier système fermé (2) est un récipient, en particulier une bouteille sertie en verre ou en plastique, un sac de culture cellulaire ou un sac de sang, ou un système fermé de complexité quelconque et est fermé par un septum (21), dans lequel le septum (21) est préférablement constitué d'au moins un élastomère et dans lequel la connexion à l'étape c) du procédé est préférablement effectuée en ponctionnant la au moins une canule (11) du connecteur stérile (1) dans le septum (21).

9. Le procédé de l'une des revendications 7 ou 8, dans lequel le deuxième système fermé (3) a un tube, en particulier un tube en plastique, en particulier un tube en PVC, par lequel le connecteur stérile (1) est connecté au deuxième système fermé (3) à l'étape d) du procédé, préférablement au moyen d'une soudure de tube stérile.
